# EUROPÄISCHE PATENTSCHRIFT

(11) **EP 2 349 359 B1**
(45) Veröffentlichungstag und Bekanntmachung des Hinweises auf die Patenterteilung: **24.07.2013**
(21) Anmeldenummer: 09760090.2
(22) Anmeldetag: 30.10.2009
(51) Int. Cl.: A61L 27/02, A61L 27/34, A61L 27/54

(54) **MEDIZINISCHES IMPLANTAT MIT BIOFUNKTIONALISIERTER OBERFLÄCHE**
MEDICAL IMPLANT HAVING BIO-FUNCTIONALIZED SURFACE
IMPLANT MÉDICAL AVEC UNE SURFACE BIOFONCTIONNALISÉE

(30) Priorität: 30.10.2008 DE 102008053892
(43) Veröffentlichungstag der Anmeldung: 03.08.2011
(73) Patentinhaber: Fachhochschule Gelsenkirchen, 45877 Gelsenkirchen (DE)
(72) Erfinder: VEITH, Michael, 45721 Haltern (DE); LEHNERT, Michael, 45657 Recklinghausen (DE); GENESIUS, Katharina, 45891 Gelsenkirchen (DE); MEYER, Gerhard, 46483 Wesel (DE); LOIDL-STAHLHOFEN, Angelika, 46483 Wesel (DE); GORBAHN, Miriam, 58452 Witten (DE)
(74) Vertreter: Bülle, Jan
(86) Internationale Anmeldenummer: PCT/EP2009/007787
(87) Internationale Veröffentlichungsnummer: WO 2010/049171

(56) Entgegenhaltungen:
- WO-A2-99/26674
- US-A1- 2007 254 006

## Beschreibung

Die Erfindung stellt ein medizinisches Implantat, insbesondere ein Dentalimplantat mit einer biofunktionalisierten Oberfläche zur Verfügung.

Das erfindungsgemäße Implantat umfasst einen Kern und ein über mindestens einen Linker verbundenes Biomolekül.

Ein entscheidender Aspekt für die Integration von Implantaten ist die Verankerung des Implantats. Dem Fachmann ist hierfür eine Vielzahl von Methoden, teilweise besonders angepasst für einzelne Implantat-Arten, bekannt. Soweit es sich dabei um Implantate handelt, die in die Nähe von Knochen eingesetzt werden, sind dem Fachmann vielerlei Ansätze zur Modifizierung der Implantats-Oberfläche bekannt, die einer Integration des Implantats in den Knochen förderlich sind.

Die geringste Form der Oberflächenmodifizierung bezieht sich dabei auf ein Anrauen oder eine Strukturierung der Implantat-Oberfläche zum verbesserten Einwachsen in den Knochen. Genannt sei an dieser Stelle beispielsweise die Schrift WO0224243 ("*Method for surface treatment of implants or prosthesis made of titanium or other materials*"), welche insbesondere Hüft- und Knie-Implantate (Prothesen) offenbart oder DE102006011629 ("Verfahren zur Herstellung von Implantaten").

Ein weiteres Verfahren zur Förderung der Integration in den Knochen ist die Einbringung bzw. Auftragung von Membranen auf das Implantat mit dem Ziel, so das Einwachsen in den Knochen zu verbessern, wie sie beispielsweise in DE19948787 ("Medizinische Membran zur Anregung der Gewebebildung"), DE19748688 ("Membransystem zur gesteuerten Geweberegeneration bei Erkrankungen des Zahnhalteapparates") und US20020133232 ("*Microstructured dual sided membrane for tissue growth and regeneration*") beschrieben werden.

Auch die Verwendung von netzartigen Strukturen aus biokompatiblen Materialien wird als Additiv zur Einheilung von Implantaten beschrieben (EP0310623B1 ("*Biocompatible particles and cloth-like article made therefrom*").

Ferner wird auch die Mineralisierung der Oberfläche durch verschiedene Beschichtungen beispielsweise Calcium-Phosphat zur Verbesserung der Integration in den Knochen offenbart (DE3905608 ("Dentalimplantat), EP1516602B1 ("*Implant with bioactive particles stuck and method of manufacturing the same*") und EP0832619B1 ("*Implant with embedded bioactive particles and method of manufacturing the same*")).

Andere Beschichtungen setzten hier auf eine nanokristalline Apatitschicht (Hydroxylapatit und Flurapatit) mit eingebetteten Peptiden, die Teilsequenzen von Proteinen der Extrazellulären Matrix (EZM) darstellen und die Osteoblastenintegration steigern sollen (WO06004778A2 ("*Implant with a biofunctionalized surface and method for its production*")).

Beschichtungen aus Polymeren werden ebenfalls häufig beschrieben. Neben vollsynthetischen Polymeren wie PMMA, die meist nur als Mittelschicht für die Anheftung weiterer Polymerschichten verwendet wird (US4812120 ("*Implantable percutaneous device*")), werden vermehrt biologische, körpereigene Biopolymere wie Kollagen, Hyaluronsäure oder aus Milch- und Glykolsäure (US646138 ("*Method and apparatus for augmenting osteointegration of prosthetic implant devices*"), EP0470305A1 ("*Osteoprosthetic implant*")) eingesetzt.

Weiteren Einsatz finden Polymere, welche sich mit der Zeit von alleine abbauen und nach Einwachsen des Implantats keine Rückstände mehr im Körper hinterlassen, sogenannte "biodegradabte Polymere" (DE60120660 ("Zusammensetzungen von vernetzbaren Prepolymeren für bioabbaubare implantate")).

Wachstumsfaktoren und Adhäsionsmoleküle kommen ebenfalls bei Implantaten zum Einsatz. Insbesondere das *Bone Morphegenetic Protein* (BMP) (US6214049 ("*Method and apparatus for augmentating osteointegration of prosthetic implant devices*")) und Proteine der EZM wie Fibronektin werden in verschiedensten Beschichtungen zur besseren bzw. schnelleren Einheilung des Implantats in den Knochen oder auch für Implantathalterungen verwendet (WO04010887A1 (*"Arrangement for using osteoinductive or bioactive material to induce bone and*/*or increase the stability of implants in the jaw bone, and an implant intended for this purpose*")).

Eine indirekte Methode, die dasselbe Ziel wie die bereits genannten Schriften verfolgt, aber keine Modifizierung der Implantatoberflächen beinhaltet, ist die Verwendung von Gelen. Diese werden entweder auf das Implantat oder während der Implantation auf das umliegende Gewebe verteilt. In dem Dokument (EP0726741 ("*Implant assembly*")) wird die Anwendung des Wachstumsfaktors *Fibroblast Growth Faktor* (FGF) in Gelen mit dem Ziel, das Zellwachstum zu fördern, beschrieben.

Neben der Langzeitintegration von Implantaten ist ein weiterer entscheidender Aspekt für den Erfolg der Implantation, die Schnelligkeit, mit der das Implantat überhaupt erst einmal in das Gewebe integriert. Dieses ist insbesondere dann entscheidend, wenn es an der Stelle der Implantation zu Infektionen kommen kann.

Ein Beispiel ist die Implantation von Dentalimplantaten. Hier kann es zum Eindringen von Bakterien kommen, die eine Periimplantitis hervorrufen können.

Zwar sind einige der im vorhergehenden beschriebenen Offenbarungen nicht nur geeignet, eine stabile Verankerung des Implantats zu gewährleisten, sondern sind auch geeignet, den Verankerungsprozess zu beschleunigen, allen gemeinsam ist jedoch, dass die dort offenbarten Lehren auf eine Verankerung des Implantats im Knochen abzielen.

Das Risiko des Auftretens einer Periimplantitis wird dadurch jedoch nicht vermindert.

Nur wenige Ansätze verfolgen das Ziel, die Fibroblastenadhäsion an das Implantat zu steigern, um dadurch die Gefahr der Periimplantitis zu verringern. Eine Methode, die wiederum auf Osteoblasten aber auch auf Peridontalfibroblasten abzielt, ist die Verwendung einer gelartigen Matrix, die direkt vor der Implantation auf die Implantatoberfläche gegeben wird. Das Gel besteht aus einem PMMA/ Kollagenverbundmaterial, das Faktoren enthält, die die Knochenhautzellen stimulieren sollen (DE19630034A1 ("Biohybrides Zahnimplantat")). Eine Möglichkeit der dort beschriebenen Beschichtung ist schichtartig aufgebaut. Grundschicht auf dem Titankem ist ein Polymer aus PMMA oder Bis-γ-Methacrylalat mit Glasfasern. Auf diese Schicht wird eine zweite Schicht aus dem vorhergehenden Polymer mit eingebetteten Kollagenfasern aufgebracht. In diese können verschieden Metabolite und Moleküle wie Wachstumsfaktoren oder Fibronektin eingelagert sein (DE3627316 ("Collagenimplantate, Verfahren zu ihrer Herstellung und Verwendung")). Ein anderer Ansatz zur Behandlung von Peridontitis wird in EP0404792 ("*A method of treating conditions of teeth and their supporting tissue*") beschrieben. Die hier eingesetzte saccharose-sulfathaltige Paste wird nachträglich auf die entzündete Stelle aufgetragen und soll zur Heilung beitragen. Ein weiterer Ansatz zur Fibroblastenadhäsion wird in WO07050130 ("*Gold surfaces coated with a thermostable, chemically resistant polypeptide layer and applications thereof*") beschrieben. Das Titanimplantat wird mit einer dünnen Goldschicht beschichtet. Auf diese Goldschicht können Fusionsproteine über Thiofunktionalisierung immobilisert werden.

Neben den oben genannten Möglichkeiten finden auch körpereigene Substanzen wie Hyaluronsäure, Chitosan und andere Anwendung in der Beschichtung von Dentalimplantaten. Chitosanfilme aus Chitosan und biologisch abbaubaren Polymeren in Kombination mit einer bioaktiven Substanz z.B. Antibiotika, Hormone, Proteine und BMP's werden zur verbesserten Zellanhaftung eingesetzt (EP1308177 ("*Method for the production of chitosan-based films with enhanced cell adhering capacity, resulting product and applications*")). Chitosan wird aber auch zur Förderung der Osseointegration als alleinige Beschichtung eingesetzt (DE69733840 ("Implantierbarer künstlicher Zahn beschichtet mit Chitosan")). Weiter werden die körpereigenen Substanzen in Verknüpfung mit Hydoxylapatit auch als Füllmaterial für Knochendefekte oder als Hilfsmittels zur Bedeckung dieser eingesetzt (DE2756256 ("Hilfsmittel zum Bedecken und/oder Ausfüllen von Knochendefekten und Verfahren zur Herstellung desselben")). Ein weiteres Biomaterial aus körpereigenen Substanzen, das Einsatz als Knochenersatz findet, wird in EP1142595 ("*Biomaterials for bone replacement*") beschrieben. Hier wird Hyaluronsäure und/oder ihre Derivate zusammen mit granulärem Knochenmaterial als Knochenersatzmaterial für Defekte eingesetzt. Einige Offenbarungen zielen darauf ab, die Gewebeverträglichkeit von Implantaten zu verbessern (DE10223310 ("Verfahren zum Beschichten von Implantaten mit einer Polysaccharid-Lage")).

Nicht nur körpereigene Substanzen werden eingesetzt, um die Gewebsverträglichkeit zu steigern. Auch synthetische Polymere oder eine Kombination aus synthetischen und biologischen Polymeren werden eingesetzt, wie in DE4444445 ("Verfahren zur Herstellung gewebeverträglicher Substrate, gewebeverträgliches Substrat und seine Verwendung") offenbart. Demgemäß wird ein Implantat durch ein Polymersubstrat mit einem hydrophilen Polymer auf synthetischer und/oder biologischer Basis mittels chemischer Verankerung modifiziert.

Die Bedeutung der Förderung der Integration des Implantats in das Zahnfleisch ist somit in der Implantat-Entwicklung bislang nur vergleichsweise wenig Beachtung geschenkt worden und wird somit bislang nur sehr ungenügend gelöst.

Das im vorher gehenden erwähnte Anrauen oder Strukturieren der Implantat-Oberfläche wirkt sich zwar positiv auf das Anwachsen von Knochengewebe aus. Auf das Bindegewebe bzw. die Gingiva wirkt eine raue Oberfläche jedoch eher störend, da Fibroblasten glatte Oberflächen bevorzugen. Aufgrund der Inertheit von (typischerweise verwendeten) TiOₓ-Oberflächen kommt es zu keinerlei chemischer Wechselwirkung zwischen Implantat und Gewebe.

Auch die aus dem Stand der Technik bekannten Membran-Beschichtungen der Implantate (s. beispielsweise die im vorgehenden zitierten Dokumente DE19948787, DE19748688 und US20020133232) sind nicht für die Integration in das Gingiva-Gewebe optimiert, sondern allenfalls an eine Integration in das Knochengewebe angepasst.

Ähnliches gilt für die bereits erwähnte Mineralisierung der Implantate.

Der bereits erwähnte Einsatz von körpereigenen Biopolymeren wie Kollagenen oder GAG's, vor allem die Hyaluronsäuren, wie in EP0470305A1 ("*Osteoprosthetic implant*"), EP0404792 ("*A method of treating conditions of teeth and their supporting tissue*"), DE10223310 ("Verfahren zum Beschichten von Implantaten mit einer Polysaccharid-Lage") oder DE69733840 ("Implantierbarer künstlicher Zahn beschichtet mit Chitosan") beschrieben, schafft eine Umgebung, welche Komponenten der Extrazellulären Matrix aufweist und so die natürliche Umgebung der Gingiva-Fibroblasten mimt. Hierdurch soll das Zellanwachsen verbessert werden. Biologische Polymere wie beispielsweise Kollagen und Hyaluronsäure sind wichtige Komponenten der EZM, allerdings haben sie strukturgebende Funktionen (Kollagen) oder verleihen der Matrix Druckfestigkeit und speichern wichtige Signalstoffe (GAGs wie Hyaluronsäure). Ohne das Vorhandensein weiterer Komponenten der Extrazellulären Matrix ist es diesen Biopolymeren nicht möglich, ein Zellanwachsen zu fördern, wie es auf natürlichem Wege über die Wachstumsfaktoren und Adhäsionsproteine erst ermöglicht wird.

Bei den extrazellulären Wachstumsfaktoren und Adhäsionsmolekülen treten vor allem Mitglieder der Familien der Bone Morphogenic Proteins (BMP's), der FGF's und Fibronektin, bzw. deren Sequenzabschnitte in den Vordergrund.

Die Verwendung von BMPs, wie offenbart in US6214049 ("*Method and apparatus for augmentating osteointegration of prosthetic implant devices*"), WO04010887A1 ("*Arrangement for using osteoinductive or bioactive material to induce bone and*/*or increase the stability of implants in the jaw bone, and an implant intended for this purpose*") oder EP1308177 ("*Method for the production of chitosan-based films with enhanced cell adhering capacity, resulting product and applications*") dient der Anregung des Knochenwachstums und damit der Integration des Implantates in den Kieferknochen, was wiederum keinen Einfluss auf das Anwachsen der Gingivafibroblasten hat und somit nicht vor dem Eindringen von Krankheitserregern in die Wundtasche und der damit möglicherweise entstehender Periimplantitis schützt.

Einen direkten Einfluss auf die Proliferation dieses Zelltyps besitzen FGF's (EP0726741 ("*Implant assembly*")). FGF ist ein wichtiges Signalprotein, welches die Proliferation von Fibroblasten beeinflusst. Die Adhäsion an Implantatoberflächen selbst beeinflussen sie jedoch nur gering. Weiterhin ist davon auszugehen, dass Fibroblasten, denen ihre extrazelluläre Umgebung über entsprechende Adhäsionsmoleküle ausreichend gut vorgetäuscht wird, autark beginnen eine EZM zu synthetisieren und ihre eigene Proliferation einleiten. Der entscheidende Schritt ist somit das Anheften dieser Zellen über Adhäsionsmoleküle.

In dem Dokument DE000003627316 ("Collagenimplantate, Verfahren zu ihrer Herstellung und Verwendung") werden Kollagenfasern verwendet. Zusätzlich werden weitere adhäsionsfördernde Substanzen berücksichtigt (wie Fibronektin). Fibronektin in seiner natürlichen Umgebung besitzt starke zelladhäsive Wirkung, sowohl bei Osteoblasten wie auch bei Fibroblasten. Voraussetzung für die biologische Aktivität ist allerdings das Vorliegen des Proteins in nativer Konformation. Bei unspezifischer Bindung auf Metalloberflächen oder Einlagerung in andere Oberflächen ist die Gefahr hoch, dass sich die Konformation ändert, was zu einem Aktivitätsverlust führen kann. Allerdings ist es äußerst fraglich, ab diese Moleküle innerhalb der Polymerschicht überhaupt ihre natürliche Konformation einnehmen können und damit ihre Funktion beibehalten. Auch werden keine weiteren Bestandteile der EZM verwendet, um das Maß der Biokompatibilität zu erhöhen.

Die Implantatbeschichtung, dargestellt in dem Dokument WO07050130 ("*Gold surfaces coated with a thermostable, chemically resistant polypeptide layer and applications thereof*"), zielt ebenfalls auf die Verbesserung der Fibroblastenadhäsion. Eingesetzt werden aber nur die RGD-Erkennungssequenzen und keine ganzen Proteine. Dies ist von Nachteil, da Fibronektin über eine Vielzahl an intramolekularen Wechselwirkungen (z.B. Wechselwirkung zwischen Typ3 Domäne 9 und 10) verfügt, die die Bindungsaffinität des Proteins weiter erhöht. Weiterhin unterdrückt die auf die Titanschicht aufgetragene Gold-Deckschicht alle bioinnerten positiven Eigenschaften eines Titanimplantats.

Wie bereits erwähnt, ist die Implantation von Implantation per se mit dem Risiko der Infektion behaftet.

Begünstigt durch eine stark ansteigende Zahl von Implantationen in der Zahnmedizin trifft dieses zunehmend auch für den Dentalbereich zu. Hier stellt das Auftreten von Periimplantitis nach wie vor ein signifikantes Problem dar (vgl. beispielsweise Lang, H. und Borgert, S. (2008) "Periimplantitis - eine therapeutische Herausforderung" Deutsche Zahnärztliche Zeitschrift 63: 158-159). Es liegen zwar verschiedene Ansätze zur Therapie der Periimplantitis vor (Behandlung mittels Antibiotika verbunden mit einer mechanischen Plaqueentfernung), aber die Ergebnisse sind oft nicht befriedigend, auch stellen sie eine Belastung für den Patienten dar. Ein besonderes Problem ist, dass das Entstehen einer Periimplantitis nach wie vor häufig zu einem Verlust des Implantats führt (Roos-Jansaker AM (2007) "Long time follow up of implant therapy and treatment of peri-implantitis" Swed. Dent. J. 188: 7-66).

Allgemein ist von Vorteil, wenn eine etwaige Beschichtung des Kerns des Implantats möglichst fest haftet. Dieses ist insbesondere deswegen von Vorteil, da hierdurch das Risiko gemindert wird, das die Beschichtung beim Einsetzen des Implantats, beispielsweise durch Scherbeanspruchung oder durch sonstige Beschädigungen, abgezogen, abgerollt oder dergleichen wird.

Auch ist allgemein von Vorteil, wenn eine etwaige Beschichtung des Oberflächenbereichs des Kerns eine hohe Gewebeverträglichkeit aufweist.

Ein unbefriedigendes Haften von etwaigen Beschichtungen bzw. eine unbefriedigende Gewebeverträgfichkeit hat sich bislang immer wieder als Problem erwiesen.

Es ist eine Aufgabe der Erfindung, Implantate zur Verfügung zu stellen, die Vorteile gegenüber den Implantaten des Standes der Technik aufweisen.

Die Implantate sollen möglichst das Risiko des Auftretens einer Entzündung am Ort der Implantation senken.

Insbesondere war es eine Aufgabe der vorliegenden Erfindung, ein Implantat zur Verfügung zu stellen, dessen Eigenschaften geeignet sind, das Risiko des Auftretens einer Periimplantitis am Ort der Implantation im Dentalbereich zu senken.

Diese Aufgabe wird durch den Gegenstand der Patentansprüche gelöst.

Die vorliegende Erfindung stellt ein medizinisches Implantat zur Verfügung, umfassend einen Kern, mindestens einen Linker L₁, mindestens eine Biotin-Komponente B₁, mindestens einen Biotin-Bindungspartner, mindestens eine Biotin-Komponente B₂, und mindestens ein Biomolekül, wobei der Linker L₁ ein proximales Ende und ein distales Ende umfasst, mit dem proximalen Ende kovalent an den Kern und mit dem distalen Enden kovalent an die Biotin-Komponente B₁ gebunden ist, wobei zwischen der Bindung des Linkers L₁ an den Kern und der Bindung des Linkers L₁ an die Biotin-Komponente B₁ durchgehend kovalente Bindungen vorliegen; die Biotin-Komponente B₂ kovalent an das Biomolekül gebunden ist; und der Biotin-Bindungspartner wenigstens eine Bindungsstelle S₁ für die Biotin-Komponente B₁ aufweist, an welche die Biotin-Komponente B₁ nicht-kovalent gebunden ist, und wenigstens eine Bindungsstelle S₂ für die Biotin-Komponente B₂ aufweist, an welche die Biotin-Komponente B₂ nicht-kovalent gebunden ist; wobei das Biomolekül ausgewählt ist aus der Gruppe bestehend aus Biopolymeren, Peptiden, Proteinen und Sacchariden.

Überraschenderweise wurde gefunden, dass der Aufbau eines solchen Implantats besonders geeignet ist, um die native Struktur des Biomoleküls zu erhalten. Durch den Erhalt der nativen Struktur wird die Wechselwirkung mit dem Gewebe am Ort der Implantation begünstigt, dass Einwachsen somit erleichtert.

Besonders wichtig ist das Vorliegen der nativen Struktur beispielsweise im Falle des Fibronektins. Dieses Protein enthält verschiedene kurze Peptidesequenzen, die für die Wechselwirkung mit den Integrinen verantwortlich sind. In Fibronektin-Molekülen, welche nicht in einer nativen Konformation vorliegen, ist eine optimale Ausrichtung dieser Peptid-Sequenzen nicht gewährleistet.

Die Verwendung von Biotin-Komponenten gewährleistet eine feste und sogleich flexible Bindung des Biomoleküls an den Kern.

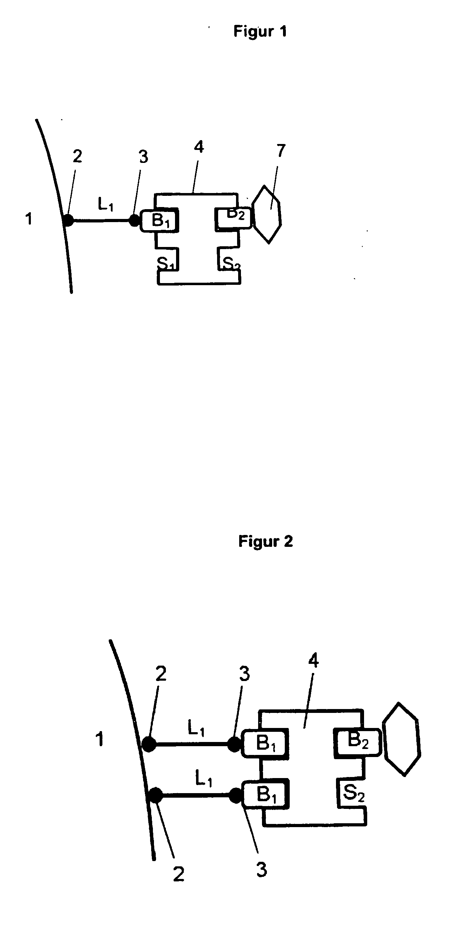

Figur 1 zeigt eine schematisierte und vereinfachte Darstellung einer bevorzugten Ausführungsform des erfindungsgemäßen Implantats. An den Kern (1) ist kovalent das proximale Ende (2) des Linkers L₁ gebunden. An das distale Ende (3) des Linkers L₁ ist kovalent die Biotin-Komponente B₁ gebunden. Zwischen der kovalenten Bindung der Linkers L₁ an den Kern (1) am proximalen Ende (2) und der kovalenten Bindung des Linkers L₁ an die Biotin-Komponente B₁ am distalen Ende (3) liegen durchgehend kovalente Bindungen vor. An das Biomolekül (5) ist kovalent die Biotin-Komponente B₂ gebunden. Der Biotin-Bindungspartner (4) weist zwei Bindungsstellen S₁ und zwei Bindungsstellen S₂ auf. An eine der beiden Bindungsstellen S₁ ist nicht-kovalent die Biotin-Komponente B₁ gebunden. An eine der beiden Bindungsstelle S₂ ist nicht-kovalent die Biotin-Komponente B₂ gebunden.

Figur 2 zeigt eine schematisierte und vereinfachte Darstellung einer bevorzugten Ausführungsform des erfindungsgemäßen Implantats. Es weist zwei benachbarte Linker L₁ auf, welche jeweils kovalent über ihr proximales Ende (2) an den Kern (1) und über ihr distales Ende (3) an jeweils eine Biotin-Komponente B₁ gebunden sind. Die beiden Biotin-Komponenten B₁ der beiden benachbarten Linker L₁ sind jeweils nicht-kovalent an eine Bindungsstelle S₁ der Biotin-Bindungsgruppe (4) gebunden.

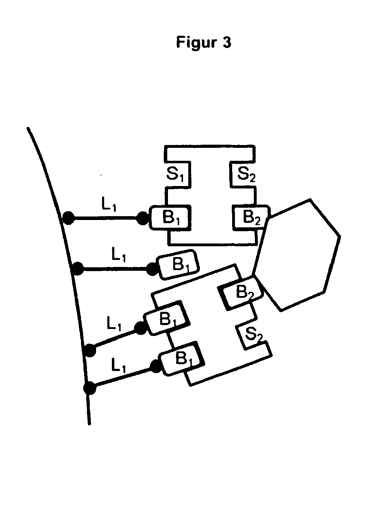

Figur 3 zeigt eine solche schematisierte und vereinfachte. Darstellung einer solchen bevorzugte Ausführungsform des erfindungsgemäßen Implantats. Es weist vier benachbarte Linker L₁ auf, welche jeweils kovalent über ihr proximales Ende an den Kern und über ihr distales Ende jeweils an eine Biotin-Komponente B₁ gebunden sind. Die Biotin-Komponenten B₁ von drei Linkem L₁ sind jeweils nicht-kovalent an eine Bindungsstelle S₁ zwei verschiedener Biotin-Bindungsgruppen gebunden. Die Biotin-Komponente B₁ des vierten Linkers ist frei. Das Biomolekül ist kovalent an zwei Biotin-Komponenten B₂ gebunden ist, welche ihrerseits nicht-kovalent an zwei Bindungsstellen S₂ von zwei verschiedenen Biotin-Bindungspartnern gebunden sind.

Figur 4-A stellt den Kern (1) dar, an den mit ihren proximalen Ende (2) der Linker L₁ gebunden ist. An das distale Ende (3) des Linkers L₁ bindet die Biotin-Komponente B₁. Die Biotin-Komponente B₁ ist vergrößert dargestellt.

Figur 4-B stellt sowohl die Linker L₁' dar, welche nicht an Biotin-Komponenten B₁ gebunden sind, als auch die Linker L₁", welche kovalent an Biotin-Komponenten B₁ gebunden sind. Die Linker L₁" binden an den Spacer (1) der Biotin-Komponente B₁, welcher an die Kopfgruppe (2) der Biotin-Komponente B₁ gebunden ist.

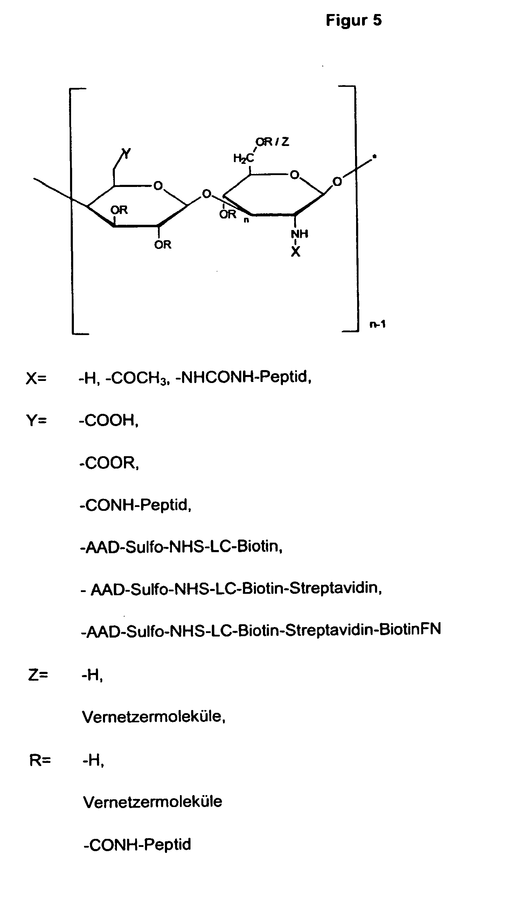

Figur 5: Hyualoronsäure-Derivat geeignet zur Verwendung in einem erfindungsgemäßen Implantat, vorzugsweise im Linker Lₚ des Polymer-Implantats

Figur 6: Chitin-Derivat geeignet zur Verwendung in einem erfindungsgemäßen Implantat, vorzugsweise im Linker Lp des Polymer-Implantats

Figur 7: Alginat-Derivat geeignet zur Verwendung in einem erfindungsgemäßen Implantat, vorzugsweise im Linker Lp des Polymer-Implantats

Figur 8: Dextran-Derivat geeignet zur Verwendung in einem erfindungsgemäßen Implantat, vorzugsweise im Linker Lp des Polymer-Implantats

Figur 9 zeigt Fibronektinadsorptionskinetiken an unterschiedlichen Titanoberflächen.

Der Begriff "medizinisches Implantat" bzw. "Implantat" im Sinne der vorliegenden Erfindung charakterisiert Materialien und Vorrichtungen, die im Zuge eines chirurgischen Eingriffes mindestens teilweise im menschlichen oder tierischen Gewebe eingebracht werden. Diese Implantate können im Kontakt zum Knochen und anderen Elementen des Stütz- und Bewegungsapparates sowie auch in Kontakt mit Blut oder Bindegewebe stehen.

Der Begriff umfasst beispielsweise Gefäßendoprothesen, intraluminale Endoprothesen, Stents, Koronarstents, periphere Stents, chirurgische bzw. orthopädische Implantate für temporäre Zwecke wie chirurgische Schrauben, Platten, Nägel und sonstige Befestigungsmittel, permanente chirurgische oder orthopädische Implantate wie Knochen-oder Gelenkprothesen, beispielsweise künstliche Hüft- oder Kniegelenke, Gelenkpfanneneinsätze, Schrauben, Platten, Nägel, implantierbare orthopädische Fixierungshilfsmittel, Wirbelkörperersatzmittel, sowie Kunstherzen und Teile davon, künstliche Herzklappen, Herzschrittmachergehäuse, Elektroden, subkutane und/oder intramuskulär einsetzbare Implantate, Wirkstoffdepots und Mikrochips, und dergleichen. In einer bevorzugten Ausführungsform handelt es sich um Katheter, welche vorzugsweise ausgewählt sind aus Blasenkathetern, Gallenkathetern und Herzkathetern. Katheter dieses Typs können insbesondere zu diagnostischen Zwecken und bei minimalinvasiven Eingriffen eingesetzt werden.

Je nach Anwendung können die Art und Eigenschaft des Biomoleküls oder der Mischung verschiedener Biomoleküle verändert werden. Ein Fachmann erkennt, dass bestimmte Implantate infolge ihrer Umgebung bestimmte Anforderungen stellen. Soll das Implantat dauerhaft im Körper verbleiben und wird dort mechanischen Einwirkungen ausgesetzt, so ist es häufig günstig, wenn das umgebende Gewebe in das Implantat einwächst oder zumindest adhäriert.

Je nach Anwendung kann es aber auch von Vorteil sein, wenn ein Zellwachstum an der Oberfläche des Implantats gerade vermieden wird.

In speziellen Ausführungen kann es sogar möglich sein, dass bestimmte Oberflächenbereiche des Implantats die Adhäsion von Zellen begünstigende Eigenschaften aufweisen, wohingegen andere Oberflächenbereiche ein und desselben Implantats die Adhäsion von Zellen behindernde Eigenschaften aufweisen. Dies kann z.B. bei Zahnimplantaten der Fall sein, bei denen ein Oberflächenbereich nach Möglichkeit mit dem Kiefer verwachsen sollte, ein anderer Oberflächenbereich mit dem Zahnfleisch verwachsen sollte und ein anderer Oberflächenbereich überhaupt nicht bewachsen werden sollte.

Bevorzugt im Sinne der vorliegenden Erfindung sind Implantate, die zum Zeitpunkt der Implantation für eine möglichst dauerhafte Implantation in das Gewebe vorgesehen sind, deren Entfernung also nicht von vornherein als Teil einer wie auch immer gearteten therapeutischen Behandlung beabsichtigt ist.

Der Begriff "Gewebe" im Sinne der vorliegenden Erfindung umfasst jedwedes Gewebe eines Organismus, vorzugsweise eines Menschen. Er umfasst beispielsweise Epithelgewebe, Binde- und Stützgewebe, Muskelgewebe, Nervengewebe und Knochengewebe. Vorzugsweise umfasst er Binde- und Stützgewebe. Der Begriff umfasst, soweit dort vorhanden, das entsprechende Gewebe in jedem beliebigen Organ und/oder Teilbereich des Körpers. Vorzugsweise umfasst er das Gewebe, vorzugsweise das Binde- und Stützgewebe, im Dentalbereich, vorzugsweise des Menschen.

Im Sinne der vorliegenden Erfindung umfasst der Begriff "medizinisches Implantat" bzw. "Implantat" insbesondere Implantate zur Implantation im tierischen oder, vorzugsweise, im menschlichen Dentalbereich (im Folgenden auch als "Dentalimplantat" bezeichnet"). Hierunter fallen beispielsweise künstliche Zahnwurzeln, die zur Befestigung von Kronen, Brücken oder Prothesen dienen, Vollimplantate, aber auch jede andere Form von Dentalimplantaten.

Implantate können aus einer Vielzahl von Materialien hergestellt werden bzw. solcherlei Materialien umfassen. Dazu gehören beispielsweise amorpher und/oder (teil-)kristalliner Kohlenstoff, Vollkarbonmaterial, poröser Kohlenstoff, Graphit, Kohlenstoffverbundmaterialien, Kohlefasern, Keramiken wie z. B. Zeolithe, Silikate, Aluminiumoxide, Aluminosilikate, Siliziumkarbid, Siliziumnitrid; Metallkarbide, Metalloxide, Metallnitride, Metallcarbonitride, Metalloxycarbide, Metalloxynitride und Metalloxycarbonitride der Übergangsmetalle wie Titan, Zirkonium, Hafnium, Vanadium, Niob, Tantal, Chrom, Molybdän, Wolfram, Mangan, Rhenium, Eisen, Kobalt, Nickel; Metalle und Metalllegierungen, insbesondere der Edelmetalle Gold, Silber, Ruthenium, Rhodium, Palladium, Osmium, Iridium, Platin; Metalle und Metalllegierungen von Titan, Zirkon, Hafnium, Vanadin, Niob, Tantal, Chrom, Molybdän, Wolfram, Mangan, Rhenium, Eisen, Kobalt, Nickel, Kupfer; Stahl, insbesondere rostfreier Stahl, Formgedächtnislegierungen wie Nitinol, Nickel-Titanlegierung, Glas, Stein, Glasfasern, Mineralien, natürliche oder synthetische Knochensubstanz, Knochenimitate auf Basis von Erdalkalimetallkarbonaten wie Kalziumkarbonat, Magnesiumkarbonat, Strontiumkarbonat, sowie geeignete Kombinationen der genannten Materialien in jeweils einer oder mehreren Phasen.

Insbesondere wegen den spezifischen Anforderungen im Dentalbereich, wie beispielsweise eine hohe Druckbelastbarkeit und eine möglichst hohe chemische Stabilität unter den Bedingungen des Dentalbereichs, sind einige Materialien für die Verwendung im Dentalbereich besonders bevorzugt.

Zu den bevorzugten Materialien für die Verwendung im Dentalimplantaten zählen beispielsweise Metalle wie Gold, Platin und Titan bzw. Verbindungen und/oder Legierungen enthaltend diese Metalle, wobei Titan als Metall bzw. seine Oxide besonders bevorzugt sind. Daneben zählen zu den bevorzugten Materialien auch Keramiken umfassend Al₂O₃ oder Keramiken umfassend Zirkonoxide. Alle diese Materialien können jeweils auch in geeigneter Weise miteinander kombiniert werden und in jeweils einer oder mehreren Phasen vorliegen.

Insbesondere kann das Implantat in seinem Inneren andere dieser und/oder anderer Materialien umfassen als in weiter außen liegenden Schichten.

Bei dem Linker L₁ kann es sich um ein lineares Molekül handeln. Es kann sich auch um ein verzweigtes Molekül handeln. Neben dem proximalen Ende können auch noch weitere Enden an den Kern gebunden sein. In einer Ausführungsform ist mindestens eines dieser mindestens einen freien Enden ebenfalls kovalent an den Kern gebunden.

Die Erfindung umfasst Implantate, umfassend Linker L₁, die am distalen Ende kovalent an die Biotin-Komponente B₁ gebunden sind. Diese Linker L₁ werden auch als Linker L₁" bezeichnet. Neben dem distalen Ende können auch noch weitere Enden an die Biotin-Komponenten B₁ gebunden sein. In einer Ausführungsform ist mindestens eines dieser mindestens einen freien Enden ebenfalls kovalent an die Biotin-Komponenten B₁ gebunden.

Linker L₁, welche nicht an eine Biotin-Komponente B₁ gebunden sind, werden auch als Linker L₁' bezeichnet.

In einer bevorzugten Ausführungsform ist das Biomolekül unmittelbar kovalent an die Biotin-Komponente B₂ gebunden, wobei die Biotin-Komponente B₂ analog zur Biotin- Komponente B₁ ebenfalls in Kopfgruppe und Spacer unterteilt werden kann.

In einer anderen bevorzugten Ausführungsform ist das Biomolekül über einen Linker L₂ an die Biotin-Komponente B₂ gebunden. Der Linker L₂ weist ein proximales Ende und ein disatles Ende auf. Bei dem Linker L₂ kann es sich um ein lineares Molekül handeln. Es kann sich auch um ein verzweigtes Molekül handeln. Es können sowohl alle Enden des Linker L₂ kovalent an die Biotin-Komponenten B₁ bzw. an das Biomolekül gebunden sein als auch frei vorliegen. Neben dem proximalen Ende können auch noch weitere Enden an die Biotin-Komponenten B₁ gebunden sein. In einer Ausführungsform ist mindestens eines dieser mindestens einen freien Enden ebenfalls kovalent an die Biotin-Komponenten B₁ gebunden. Neben dem distalen Ende können auch noch weitere Enden das Biomolekül gebunden sein. In einer Ausführungsform ist mindestens eines dieser mindestens einen freien Enden ebenfalls kovalent an das Biomolekül gebunden.

Der Begriff "proximal" bzw. "proximales Ende" oder dergleichen im Sinne der vorliegenden Erfindung bezeichnet das Ende eines Moleküls, das in dem erfindungsgemäßen Implantat, im Vergleich zum distalen Ende des Moleküls, dem Kern zugewandter ist.

Der Begriff "distal" bzw. "distales Ende" oder dergleichen im Sinne der vorliegenden Erfindung bezeichnet das Ende eines Moleküls, das in dem erfindungsgemäßen Implantat, im Vergleich zum proximalen Ende, vom Kern abgewandt liegt.

Der Begriff "durchgehend kovalent" im Sinne der vorliegenden Erfindung bedeutet, dass zwischen den verbundenen Moleküle eine, zumindest gedanklich lineare, Kette von Atomen mit einem proximalen und einem distalen Ende zu finden ist, in dem die jeweiligen Atome mit dem jeweils proximal liegenden Atom und dem distal liegenden Atom durch eine kovalente Bindung verbunden ist.

In einer bevorzugten Ausführungsform liegen zwischen dem proximal liegenden Atom und dem distal liegenden Atom ausschließlich kovalente Bindungen vor.

Die Biotin-Komponente im Sinne der vorliegenden Erfindung umfasst eine Kopfgruppe.

Der Begriff "Kopfgruppe" umfasst tetrahydro-1H-thieno[3,4-d]imidazol-2(3H)-on sowie alle Varianten, Derivate und dergleichen, die mit einer Assoziationskonstante von mindestens 10⁸ M⁻¹, bevorzugter mindestens 10⁹ M⁻¹, noch bevorzugter mindestens 10¹⁰ M⁻¹, besonders bevorzugt mindestens 10¹² M⁻¹, ganz besonders bevorzugt mindestens 10¹⁴ M⁻¹ an ein oder mehrere der Moleküle Avidin (Accession numbers NM_205320.1 / GI:45384353), Neutravidin oder Streptavidin (Accession numbers CAA00084.1 / GI:14606) binden.

Weiterhin umfasst die Biotin-Komponente einen Spacer. Das distale Ende des Spacers ist mit der Ringstruktur des Biotins verbunden.

In dem erfindungsgemäßen Implantat ist das proximale Ende des Spacer der Biotin-Komponente B₁ unmittelbar kovalent verbunden mit dem distalen Ende des Linkers L₁.

In einer besonderen Ausführungsform ist das Implantat daher dadurch gekennzeichnet, dass die Biotin-Komponente einen Spacer umfasst, welcher kovalent an das distale Ende des Linkers L₁ gebunden ist.

Der Begriff "Spacer" umfasst ein organisches Molekül.

Ein bevorzugter Spacer ist eine Molekülsubstruktur gemäß der allgemeinen Formel 1

X-(CR₂)ₚ-CO-[NR-(CR₂)_{q}-CO)_{z}-L

X bezeichnet die Kopfgruppe der Biotin-Komponente.

L bezeichnet den Linker.

p = 1, 2, 3, 4, 5, 6, 7 oder 8, vorzugsweise 4

q = 1, 2, 3, 4, 5, 6, 7 oder 8, vorzugsweise 5

z = 0, 1 oder 2, vorzugsweise 0 oder 1, besonders bevorzugt 1

R = jeder Rest R ist unabhängig voneinander. Vorzugsweise ist jeder Rest R unabhängig voneinander ausgewählt aus der Gruppe bestehend aus H und organischen Molekülen.

Besonders bevorzugt ist jeder Rest R unabhängig voneinander ausgewählt aus der Gruppe bestehend aus H; Alkyl, vorzugsweise C₁-C₆-Alkyl, besonders bevorzugt ausgewählt aus der Gruppe bestehend aus CH₃, C₂H₅, i-C₃H₇, i-C₄H₉, n-C₄H₉, sec-C₄H₉, tert-C₄H₉; Alkenyl ausgewählt aus der Gruppe bestehend aus C₂H₃, i-C₃H₅, n-C₃H₅, n-C₄H₇, i-C₄H₇, sec-C₄H₇; wobei die Alkyl und Alkenyl-Gruppen einen oder mehreren Substituenten der Gruppe bestehend aus F, Cl, Br, I, CF₃, gesättigte, ungesättigte oder aromatische 3er bis 7er Ringe tragen können, optional mit einem oder mehreren Heteroatomen ausgewählt aus der Gruppe bestehend aus N, S, O. Wenn mehrere Heteroatome in einem Ring sind, können sie gleich oder unterschiedlich sein. In einer ebenfalls besonders bevorzugten Ausführungsform sind zwei beliebige Reste R Teil eines Ringes, wobei dieser Ring gesättigt oder ungesättigt sein kann und 3 bis 5 C-Atome tragen kann und optional ein oder mehrere Heteroatome ausgewählt aus der Gruppe bestehend aus N, S und O. Wenn mehrere Heteroatome in einem solchen Ring sind, können sie gleich oder unterschiedlich sein.

Ganz besonders bevorzugt ist R = H.

In einer Ausführungsform des erfindungsgemäßen Implantats beträgt der Abstand zwischen Biotin-Kopfgruppe und der Oberfläche des Kerns 0,8 bis 2 nm.

In einer besonderen Ausführungsform ist auch die Kopfgruppe der Biotin-Komponente B₂ mit einem erfindungsgemäßen Spacer verbunden.

In einer bevorzugten Ausführungsform ist das erfindungsgemäße Implantat, dadurch gekennzeichnet, dass die Biotin-Komponente B₁ an die Bindungsstelle S₁ des Biotin-Bindungspartners und/oder die Biotin-Komponente B₂ an die Bindungsstelle S₂ des Biotin-Bindungspartners über eine nicht-kovalente Wechselwirkung mit einer Assoziationskonstante von mindestens 10⁸ M⁻¹ gebunden ist.

In besonders bevorzugten Ausführungsformen ist die Biotin-Komponente B₁ an die Bindungsstelle S₁ des Biotin-Bindungspartners und/oder die Biotin-Komponente B₂ an die Bindungsstelle S₂ des Biotin-Bindungspartners über eine nicht-kovalente Wechselwirkung mit einer Assoziationskonstante von mindestens 10⁹ M⁻¹, noch bevorzugter mindestens 10¹⁰ M⁻¹, besonders bevorzugt mindestens 10¹² M⁻¹, ganz besonders bevorzugt mindestens 10¹⁴M⁻¹ gebunden.

Das erfindungsgemäße Implantat weist somit, bedingt durch die Assoziationskonstante der nicht-kovalenten Wechselwirkung, eine hohe Stabilität auf. Durch den "Baukasten"-artigen Schichtaufbau weist es außerdem eine hohe Flexibilität auf.

Geeignete Biotin-Bindungspartner sind dem Fachmann bekannt und/oder durch einfache, dem Fachmann bekannte Bindungsassays identifizierbar.

Eine besonders geeignete Kombination ist die Kombination zwischen einer Biotin-Komponente und einer Avidin-Komponente oder Neutravidin-Komponenten oder Streptavidin-Komponente.

In einer bevorzugten Ausführungsform ist somit der Biotin-Bindungspartner ausgewählt aus der Gruppe bestehend aus Avidin-, Neutravidin- und Streptavidin-Komponenten.

Der Begriff "Avidin-Komponente" im Sinne der vorliegenden Erfindung bezieht sich auf das Avidin-Protein aus Gallus gallus (Accession numbers NM_205320.1 / GI:45384353). Der Begriff umfasst weiterhin alle Orthologe dieses Proteins. Auch umfasst sind Homologe dieses Proteins bzw. des jeweiligen Orthologs dieses Proteins mit einem Homologiegrad von mindestens 70 Prozent, bevorzugt mindestens 80 Prozent, noch bevorzugter mindestens 90 Prozent, besonders bevorzugt mindestens 95 Prozent, ganz besonders bevorzugt mindestens 98 Prozent, die mit einer Assoziationskonstante von mindestens 10⁸ M⁻¹, bevorzugter mindestens 10⁹ M⁻¹, noch bevorzugter mindestens 10¹⁰ M⁻¹, besonders bevorzugt mindestens 10¹² M⁻¹, ganz besonders bevorzugt mindestens 10¹⁴ M⁻¹ an ein oder mehrere Moleküle des Biotins entsprechend der PubChem-Nummer 171548 binden. Ebenfalls umfasst sind Fragmente die mit mindestens den genannten Assoziationskonstanten an Biotin entsprechend der PubChem-Nummer 171548 binden. Ebenfalls umfasst sind chemisch modifizierte Varianten dieser Proteine oder Fragmente, die mit mindestens den genannten Assoziationskonstanten an Biotin entsprechend der PubChem-Nummer 171548 binden. Die chemisch modifizierten Varianten können sich beispielsweise durch den Grad und/oder Art der Glykosylierung unterscheiden. Umfasst sind die Fragmente, welche aufgeführt sind in Hiller et al. (1991) Biochem J. 278: 573-85.

Auch umfasst sind die gänzlich deglykosylierten Varianten dieser Proteine und Fragmente, insbesondere Neutravidin.

Der Begriff "Streptavidin-Komponente" im Sinne der vorliegenden Erfindung bezieht sich auf das Streptavidin-Protein aus Streptomyces avidinii (Accession numbers CAA00084.1 / GI:14606). Weiterhin umfasst sind alle Orthologe, Homologe, Fragmente und chemisch modifizierten Varianten analog der Definition des Begriffes "Avidin".

Streptavidin ist ein ca. 53.000 Da großes, tetrameres Protein, aus dem Aktinobakterium Streptomyces avidinii. Seine Abmessungen betragen ca.: 4,6 nm * 4,2 nm * 4,2 nm. Es fungiert als natürliches Antibiotikum, welches der Umgebung das für ein Überleben von Mikroorganismen essentielle Vitamin H entzieht.

Die Verwendung einer Streptavidin-Komponente, bevorzugt Streptavidin, als Biotin-Bindungspartner kann somit hinsichtlich der Vermeidung einer Entzündung, insbesondere einer Periimplantitis am Ort der Implantation von Vorteil sein.

Streptavidin besteht aus vier identischen Untereinheiten. Jede besitzt eine Bindungsstelle für das Molekül Biotin (Vitamin H). Dieses wird von Streptavidin hoch spezifisch über die stärkste, nicht kovalenten Wechselwirkung, die in der Natur vorkommt, gebunden (Kd = 10⁻¹⁵).

Weitere spezifische Beispiele von Avidin-Komponenten bzw. Streptavidin-Komponenten bzw. Neutravidin-Komponenten sind dem Fachmann bekannt.

Beispiele sind unter anderem aufgeführt in der Protein-Datenbank des National Center for Biotechnology Information (www.ncbi.nlm.nih.gov). Weitere Beispiele sind die Avidin-Mutanten beschrieben in WO05047317A1 ("Avidin mutants"), die Avidin-Like Proteins beschrieben in WO06045891A1 ("Avidin-like proteins from symbiotic bacteria"), das rekombinante Avidin beschrieben in WO0198349A2 ("*Recombinant avidin and its use in biotin binding*")*,* die Avidin-Derivate beschrieben in WO0027814A ("*Avidin derivatives and uses thereof*")*,* die Avidin-compositions beschrieben in WO971743A1 ("*Stable avidin composition and methods using same*"), die Streptavidin-Monomere beschrieben in WO06084388A1 ("*Monomeric streptavidin muteins*"), die modifizierten Streptavidin-Dimere beschrieben in WO06058226A2 ("*Modified dimeric streptavidins and uses thereof*"), die Biotin-binding-proteins beschrieben in WO04018509A1 ("*improved mutants of biotin binding protein*"), das dimere Streptavidin beschrieben in WO0196530A2 (" Dimeric streptavidins"), die Streptavidin-Mutanten beschrieben in WO0011152A1 ("*Streptavidin mutants having secondary functional domains*"), das "*multiflavor-Streptavidin*" beschrieben in WO9840396A1 ("MULTIFLAVOR STREPTAVIDIN"), die modifizierten Avidin- und Streptavidin-Moleküle beschrieben in WO9700329A2 ("*Modified avidin and streptavidin molecules and use thereof*")*,* das modifizierte Avidin und Streptavidin beschrieben in WO9640761A1 ("*Modified avidin and streptavidin and methods of use thereof*"), die Streptavidin-Mutanten beschrieben in WO9711183A1 ("*Streptavidin mutants*"), das Modified-Affinity Streptavidin beschrieben in WO9624606A1 ("*Modified-affinity streptavidin*") und das Recombinant Core Streptavidin beschrieben in WO9309144A1 ("*Recombinant core-streptavidin*"). Auch dieses sind Avidin-Komponenten bzw. Streptavidin-Komponenten bzw. Neutravidin-Komponenten im Sinne der vorliegenden Erfindung.

Verschiedene Varianten werden auch kommerziell vertrieben. Beispiele hierfür sind Extravidin (Sigma-Aldrich), NeutrAvidin (Thermo Scientific), NeutrAvidin (Invitrogen) und NeutraLite (Belovo). Auch dieses sind Avidin-Komponenten bzw. Streptavidin-Komponenten bzw. Neutravidin-Komponenten im Sinne der vorliegenden Erfindung.

Neben der Stärke besitzt die Biotin-Streptavidin-Bindung noch weitere Eigenschaften, die sie für die Bionanotechnologie interessant machen. So wird lediglich die Imidazolstruktur des Biotins gebunden, nicht jedoch die Carboxylgruppe. Diese steht für weitere Modifizierungen zur Verfügung. Es lassen sich beliebige Moleküle oder Proteine an Streptavidin koppeln, die über eine Biotinmodifizierung verfügen. Die vier Bindestellen liegen weiterhin in tetraedischer Form am Protein vor. Dies bewirkt, dass ein Streptavidin bis zu vier Biotine binden bzw. biotinmodifizierte Moleküle verbinden kann. So lassen sich biologische nanoskalige Architekturen generieren. Streptavidin kann auf diese Weise auch über ein Biotinmolekül mit entsprechender Ankergruppe auf metallischen Oberflächen immobilisiert werden. Bei Titan wären Phosphat und Silangruppen geeignet. (J. Spinke: Spezifische Proteinbindung an funktionalisierte, selbstorganisierte Adsorptionsschichten. Dissertation, Johannes Gutenberg Universität Mainz; 1992; S. Busse: Untersuchung molekularer Erkennungsreaktionen mit einem integriert-optischem Mach-Zehnderiterferometer. Dissertation, Johannes Gutenberg Universität Mainz; 2000).

Figur 1 zeigt eine solche schematisierte und vereinfachte Darstellung einer bevorzugten Ausführungsform des erfindungsgemäßen Implantats. An den Kern (1) ist kovalent das proximale Ende (2) des Linkers L₁ gebunden. An das distale Ende (3) des Linkers L₁ ist kovalent die Biotin-Komponente B₁ gebunden. Zwischen der kovalenten Bindung der Linkers L₁ an den Kern (1) am proximalen Ende (2) und der kovalenten Bindung des Linkers L₁ an die Biotin-Komponente B₁ am distalen Ende (3) liegen durchgehend kovalente Bindungen vor. An das Biomolekül (5) ist kovalent die Biotin-Komponente B₂ gebunden. Der Biotin-Bindungspartner (4) weist zwei Bindungsstellen S₁ und zwei Bindungsstellen S₂ auf. An eine der beiden Bindungsstellen S₁ ist nicht-kovalent die Biotin-Komponente B₁ gebunden. An eine der beiden Bindungsstellen S₂ ist nicht-kovalent die Biotin-Komponente B₂ gebunden.

In einer Ausführungsform sind die Bindungsstellen S₁ und S₂ identisch. Sie können aber auch unterschiedlich sein.

Es wurde im Rahmen der vorliegenden Erfindung gefunden, dass es von Vorteil ist, wenn ein Biotin-Bindungspartner jeweils gleichzeitig mindestens zwei Biotin-Komponenten B₁ binden kann.

Insbesondere die Verwendung von Biotin-Bindungspartner, welche über insgesamt mindestens drei, vorzugsweise vier Bindungsstellen für Biotin-Komponenten verfügen ist daher von Vorteil.

In einer bevorzugten Ausführungsform ist das erfindungsgemäße Implantat daher dadurch gekennzeichnet, dass zwei Biotin-Komponenten B₁ an zwei Bindungsstellen S₁ eines einzelnen Biotin-Bindungspartners gebunden sind.

Figur 2 zeigt eine solche schematisierte und vereinfachte Darstellung einer solchen bevorzugte Ausführungsform des erfindungsgemäßen Implantats. Es weist zwei benachbarte Linker L₁ auf, welche jeweils kovalent über ihr proximales Ende (2) an den Kern (1) und über ihr distales Ende (3) an jeweils eine Biotin-Komponente B₁ gebunden sind. Die beiden Biotin-Komponenten B₁ der beiden benachbarten Linker L₁ sind jeweils nicht-kovalent an eine Bindungsstelle S₁ der Biotin-Bindungsgruppe (4) gebunden.

Ein solchermaßen aufgebautes Implantat bietet u.a. eine hohe Flexibilität, ist aber auch besonders stabil.

Insbesondere die Verwendung der Tetramere der Avidin-, Neutravidin- oder Streptavidin-Komponenten im erfindungsgemäßen Implantat ist von Vorteil.

In einem solchen Tetramer können gleichzeitig beide Bindungsstellen S₁ an Biotin-Komponenten B₁ als auch beide Bindungsstellen S₂ an Biotin-Komponenten B₂ binden, wodurch eine hohe Stabilität des erfindungsgemäßen Implantats gewährleistet wird.

Von Vorteil ist es auch, wenn das Biomolekül über mehrere nicht-kovalente Wechselwirkungen mit einer Assoziationskonstante von mindestens 10⁸ M⁻¹, bevorzugter mindestens 10⁹ M⁻¹, noch bevorzugter mindestens 10¹⁰ M⁻¹, besonders bevorzugt mindestens 10¹² M⁻¹, ganz besonders bevorzugt mindestens 10¹⁴ M⁻¹ an die Biotin-Bindungspartner gebunden ist.

In einer bevorzugten Ausführungsform ist das erfindungsgemäße Implantat, somit dadurch gekennzeichnet, dass das Biomolekül zwei kovalent gebundene Biotin-Komponenten B2 umfasst, welche jeweils nicht-kovalent an Biotin-Bindungspartner gebunden sind.

Auch dieses dient der Erhöhung der Stabilität.

In einer Ausführungsform sind die beiden Biotin-Komponenten B₂ jeweils an das gleiche Biotin-Bindungspartner-Molekül gebunden. In einer anderen Ausführungsform sind die beiden Biotin-Komponenten B₂ jeweils an verschiedene Biotin-Bindungspartner-Moleküle gebunden.

Figur 3 zeigt eine solche schematisierte und vereinfachte Darstellung einer solchen bevorzugte Ausführungsform des erfindungsgemäßen Implantats. Es weist vier benachbarte Linker L₁ auf, welche jeweils kovalent über ihr proximales Ende an den Kern und über ihr distales Ende jeweils an eine Biotin-Komponente B₁ gebunden sind. Die Biotin-Komponenten B₁ von drei Linkem L₁ sind jeweils nicht-kovalent an eine Bindungsstelle S₁ zwei verschiedener Biotin-Bindungsgruppen gebunden. Die Biotin-Komponente B₁ des vierten Linkers ist frei. Das Biomolekül ist kovalent an zwei Biotin-Komponenten B₂ kovalent gebunden, welche ihrerseits nicht-kovalent an zwei Bindungsstellen S₂ von zwei verschiedenen Biotin-Bindungspartnern gebunden sind.

In einer bevorzugten Ausführungsform ist das Implantat dadurch gekennzeichnet, dass es eine Vielzahl von Biotin-Komponenten B₁ und eine Vielzahl von Biotin-Bindungspartnern mit Bindungsstellen S₁ und S₂ umfasst, wobei die Biotin-Komponenten B₁ jeweils über Linker L₁ kovalent an den Kern gebunden sind und zumindest ein Anteil der Biotin-Komponenten B₁ nicht-kovalent an Bindungsstellen S₁ gleicher oder verschiedener Biotin-Bindungspartner gebunden ist.

Das Ausmaß und die Verteilung des Bereichs der Oberfläche, an den erfindungsgemäße Linker L₁ kovalent gebunden ist, kann variieren. Es kann sowohl an die gesamte Oberfläche als auch nur an Teile der Oberfläche Linker L₁ kovalent gebunden sein.

In einer Ausführungsform ist im Wesentlichen nur an den Teil der Oberfläche Linker L₁ kovalent gebunden, welcher zur Verankerung in dem Gewebe, vorzugsweise dem Gingiva-Gewebe, vorgesehen ist.

Auch hier kann sich die Dichte der Biotin-Komponenten B₁ in verschiedenen Bereichen des Implantats unterscheiden.

Beispielsweise kann der Bereich des Implantats, der nicht für einen Kontakt mit Gewebe vorgesehen ist, im Wesentlichen frei von der Biotin-Komponente B₁ sein.

Andere Bereiche des Implantats können unterschiedliche Funktionalisierungen mit unterschiedlichen Biomolekülen aufweisen, beispielsweise in Abhängigkeit davon, für welches Gewebe (Gingiva, Knochengewebe oder dergleichen) sie vorgesehen sind bzw. ob zelladhäsive Wirkung oder anti-zelladhäsive Wirkung erwünscht ist (z.B. kann an bestimmten Bereichen des Implantats Gingiva-Anwachsen absolut unerwünscht sein).

Beispielsweise können Dentalimplantate sowohl für den Kontakt mit Knochengewebe als auch mit Gingiva-Gewebe vorgesehen sein. In einer besonderen Ausführungsform ist das erfindungsgemäße Implantat dadurch gekennzeichnet, dass es mindestens einen Bereich umfasst, in dem die mittlere Flächenkonzentration der Gesamtheit aller Biotin-Komponenten B₁ im Bereich von 1 - 10, vorzugsweise von 1 - 8, noch bevorzugter von 1 - 4, besonders bevorzugt von 1 - 2, ganz besonders bevorzugt von 2 Biotin-Komponenten B₁ pro 24 nm² liegt.

Vorzugsweise handelt es sich bei diesem Bereich um eine Fläche von mindestens 1,0 mm², noch bevorzugter um eine Fläche von mindestens 2,0 mm², noch bevorzugter um eine Fläche von mindestens 30 mm², ganz besonders bevorzugter um eine Fläche von mindestens 5,0 mm².

Erfindungsgemäß kann das molare Verhältnis von Biotin-Komponenten B₁ zu gebundenen Biotin-Bindungspartnern variiert werden.

In einer besonderen Ausführungsform ist das erfindungsgemäße Implantat dadurch gekennzeichnet, dass das relative molare Verhältnis von Biotin-Bindungspartnern zu Biotin-Komponenten B₁ im Bereich von 1 : 2 bis 1 : 20, vorzugsweise im Bereich von 1 : 2 bis 1 : 10, besonders bevorzugt im Bereich von 1 : 2 bis 1 : 8, ganz besonders bevorzugt im Bereich von 1 : 2 bis 1 : 4 liegt.

Dem Fachmann sind entsprechende Verfahren zur Bestimmung dieses molaren Verhältnisses bekannt.

Ein geeignetes Messverfahren ist die SPR-Spektroskopie mit Kretschmann Konfiguration. Bei einer Biotin-Komponente B1 mit einem Spacer mit den Werten p = 4 und q = 5 wird der Schichtdickenzuwachs in destilliertem Wasser bestimmt. Angenommen wird für diese Biotinkomponente ein Brechungsindex n = 1,5. Nach der Messung wird über einen Fresnell-Fit die Schichtdicke bestimmt. Diese sollte bei 0,1 nm - 0,4 nm liegen, besonders bevorzugt bei 0,1 - 0,2 nm. Bei einer anderen Biotinkomponente B1 mit einem Spacer der allgemeinen Formel 1 mit p = 4, q = 5 und z = 2 wird in Ethanol gemessen und nach dem Fresnell-Fit sollte die Schichtdicke 0,1 nm - 0,7 nm betragen, besonders bevorzugt 0,1 nm - 0,3 nm.

Ein Verhältnis von 1 : 2 bedeutet hierbei, dass sämtliche Biotin-Komponenten B₁ einen Biotin-Bindungspartner enthaltend zwei Bindungsstellen S₁ binden.

Auch das Verhältnis von Biomolekülen zu Biotin-Bindungspartnern kann variiert werden.

Dadurch kann die Dichte der Biomoleküle auf dem erfindungsgemäßen Implantat variiert werden und somit beispielsweise an bestimmte Gewebe angepasst werden.

In einer besonderen Ausführungsform ist das erfindungsgemäße Implantat daher dadurch gekennzeichnet, dass es verschiedene Oberflächenbereiche mit einer Mindestfläche von jeweils 1,0 mm² umfasst, an welche verschiedene Biomoleküle oder verschiedene Mischungen unterschiedlicher Biomoleküle gebunden sind.

Dem Fachmann ist bekannt, welche Mischung an Biomoleküle für welche Oberflächenbereiche (beispielsweise Oberflächenbereiche die für den Kontakt mit Knochengewebe oder Gingiva-Gewebe vorgesehen sind) besonders geeignet sind.

In einer besonderen Ausführungsform beträgt die mittlere gemessene Streptavidinschichtdicke mindestens 3 nm - 4,4 nm, besonders bevorzugt 3,8 nm - 4,2 nm, ganz besonders bevorzugt 4 nm. Die Messung der "Schichtdicke" erfolgt dabei bevorzugt durch SPR-Spektroskopie (*Surface Plasmon Resonance Spectroscopy*) und anschließenden Fresnel-Fit. Der angenommene Brechungsindex für Streptavidin beträgt hierbei n = 1,45. Gemessen wird in PBS, NaCl oder vergleichbaren Lösungen. Für das Biomolekül, vorzugsweise für Fibronektin, liegt die mittlere gemessene Schichtdicke bevorzugt zwischen 1,5 - 7 nm, besonders bevorzugt 3 - 6 nm, ganz besonders bevorzugt 4,5 nm. Der angenommene Brechungsindex für Fibronektin beträgt n = 1,4. wird in PBS, NaCl oder einer vergleichbaren Lösung.

Es hat sich gezeigt, dass ein wichtiger Aspekt für die Bindung des Biotin-Bindungspartners an die Biotin-Komponente B₁ die Zugänglichkeit der Biotin-Komponente B₁ ist.

Weiterhin hat sich im Rahmen der Erfindung gezeigt, dass es wesentlich für die Zugänglichkeit der Biotin-Komponente B₁ ist, dass ein gewisser Abstand zwischen der Biotin-Komponente B₁ und anderen Molekülen vorliegt.

In einer besonderen Ausführungsform ist das erfindungsgemäße Implantat somit dadurch gekennzeichnet, dass es Linker L₁' enthält, welche nicht an Biotin-Komponenten B₁ gebunden sind, und Linker L₁" enthält, welche kovalent an Biotin-Komponenten B₁ gebunden sind, wobei der mittlere Abstand (theoretische Länge) der distalen Enden der Linker L₁" zum Schwefelatom der Biotin-Komponenten B₁ vorzugsweise zwischen 0,5 und 5,0 nm, besonders bevorzugt zwischen 0,9 und 4 nm, noch bevorzugter zwischen 1,4 und 2,7 nm, noch bevorzugter zwischen 1,4 und 2,0 nm ganz besonders bevorzugt 1,6 nm beträgt.

Der Begriff "Schwefelatom der Biotin-Komponenten" im Sinne der vorliegenden Erfindung bezieht sich auf das Schwefelatom in der Ringstruktur der Biotin-Kopfgruppe.

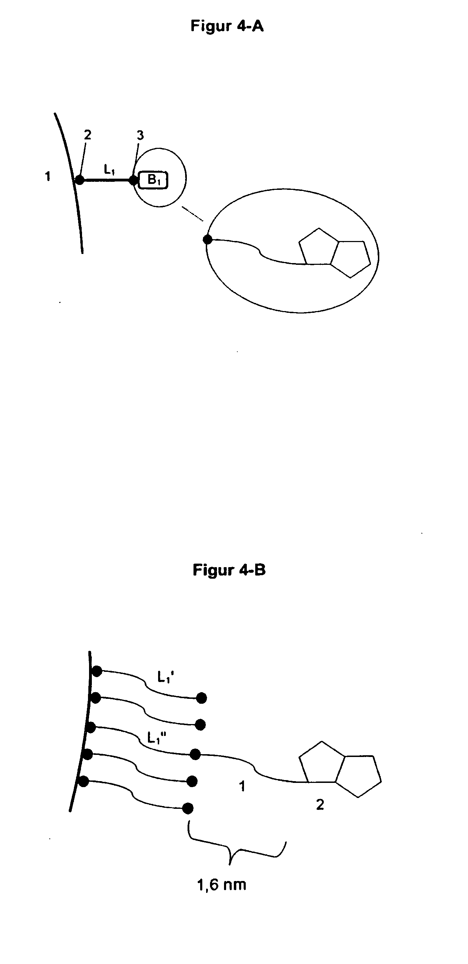

Figur 4 illustriert eine solche schematisierte und vereinfachte Darstellung einer solchen bevorzugte Ausführungsform des erfindungsgemäßen Implantats.

Figur 4-A stellt den Kern (1) dar, an den mit seinem proximalen Ende (2) der Linker L₁ gebunden ist. An das distale Ende (3) des Linkers L₁ bindet die Biotin-Komponente B₁. Die Biotin-Komponente B₁ ist vergrößert dargestellt.

Figur 4-B stellt sowohl die Linker L₁' dar, welche nicht an Biotin-Komponenten B₁ gebunden sind, als auch die Linker L₁", welche kovalent an Biotin-Komponenten B₁ gebunden sind. Die Linker L₁" binden an den Spacer (1) der Biotin-Komponente B₁, welcher an die Kopfgruppe (2) der Biotin-Komponente B₁ gebunden ist.

Das proximale Ende des Linker L₁ kann auf unterschiedliche Art und Weise kovalent an den Kern gebunden werden.

In einer bevorzugten Ausführungsform ist das proximale Ende des Linkers L₁ über mindestens ein Si- oder P-Atom an den Kern gekoppelt.

Das Si- oder P-Atom kann Teil einer funktionellen Gruppe sein. Besonders bevorzugte funktionelle Gruppen sind Silane, Siloxane, Phosphatene, Phosphonate und Phosphorane.

Somit ist eine bevorzugte Ausführungsform des erfindungsgemäßen Implantats dadurch gekennzeichnet, dass das Si- oder P-Atom Teil einer funktionellen Gruppe ist ausgewählt aus Silanen, Siloxanen, Phosphaten, Phosphonaten und Phosphoranen.

In einer bevorzugten Ausführungsform sind diese funktionellen Gruppen terminal funktionalisiert.

Die Funktionalisierung kann beispielsweise erfolgen, wie nachfolgend beschrieben:

Die Beschichtung der Titandioxidschicht kann über Chemiesorption erfolgen. Die kovalente Anbindung und Aktivierung der Titanschicht zur weiteren Modifizierung erfolgt über terminal funktionalisierte Silane oder terminal funktionalisierte Phosphonate. Die Funktionalisierung kann durch Amino-, Hydroxy, Carboxy-, Epoxid-, Vinyl-Gruppen oder eine Radikalinitiator-Gruppe wie ein Derivat des Benzoylperoxids oder des 2,2'Azo-bis-Isobytyronitrils erfolgen.

Hierbei werden bevorzugt folgende Molekülstrukturen der allgemeinen Formel 2 verwendet:

X-[{(CR₂)ₚ-NR}_{q}-(CR₂)ₒ]-NH₂

Mit p = 1, 2, 3, 4, 5, 6, 7, 8, 9 oder 10, bevorzugt 2, 3 oder 4, besonders bevorzugt 3;
q = 0, 1 oder 2; bevorzugt 1;
o = 1, 2, 3, 4, 5, 6, 7, 8, 9, 10, 11, 12, 13, 14, 15, 16, 17, 18, 19, 20, 21, 22, 23, 24, 25, bevorzugt 3, 4, 5, 6, 7, 8, 9 oder 10, besonders bevorzugt 5, 6, 7, ganz besonders bevorzugt 6.
X bezeichnet die Ankergruppe, durch die die kovalente Bindung zur Titanoberfläche hergestellt wird und ist vorzugsweise ausgewählt aus der Gruppe bestehend aus Silanen, Siloxanen, Phosphatenen, Phosphonaten und Phosphoranen.
R = jeder Rest R ist unabhängig voneinander. Vorzugsweise ist jeder Rest R unabhängig voneinander ausgewählt aus der Gruppe bestehend aus H und organischen Molekülen.

Besonders bevorzugt ist jeder Rest R unabhängig voneinander ausgewählt aus der Gruppe bestehend aus H; Alkyl, vorzugsweise C₁-C₆-Alkyl, besonders bevorzugt ausgewählt aus der Gruppe bestehend aus CH₃, C₂H₅, i-C₃H₇, i-C₄H₉, n-C₄H₉, sec-C₄H₉, tert-C₄H₉; Alkenyl ausgewählt aus der Gruppe bestehend aus C₂H₃, i-C₃H₅, n-C₃H₅, n-C₄H₇, i-C₄H₇, sec-C₄H₇; wobei die Alkyl und Alkenyl-Gruppen einen oder mehreren Substituenten der Gruppe bestehend aus F, Cl, Br, I, CF₃, gesättigte, ungesättigte oder aromatische 3er bis 7er Ringe tragen können, optional mit einem oder mehreren Heteroatomen ausgewählt aus der Gruppe bestehend aus N, S, O. Wenn mehrere Heteroatome in einem Ring sind, können sie gleich oder unterschiedlich sein. In einer ebenfalls besonders bevorzugten Ausführungsform sind zwei beliebige Reste R Teil eines Ringes, wobei dieser Ring gesättigt oder ungesättigt sein kann und 3 bis 5 C-Atome tragen kann und optional ein oder mehrere Heterotatome ausgewählt aus der Gruppe bestehend aus N, S und O. Wenn mehrere Heteroatome in einem solchen Ring sind, können sie gleich oder unterschiedlich sein.

Ganz besonders bevorzugt ist R = H.

In einer Ausführungsform des erfindungsgemäßen Implantats umfasst der Linker L1 eine Molekülstruktur der allgemeinen Formel 2. In einer bevorzugten Ausführungsform des erfindungsgemäßen Implantats besteht der Linker L1 aus einer Molekülstruktur der allgemeinen Formel 4.

Des Weiteren stellt die vorliegende Erfindung ein medizinisches Implantat zur Verfügung, umfassend einen Kern, einen Linker Lₚ und ein Biomolekül, wobei der Kern einen Oberflächenbereich aus einem anorganischen Material umfasst; der Linker Lp ein proximales Ende aufweist, das aus einem Polymer gebildet wird, welches den Oberflächenbereich kontaktiert; und das Biomolekül ausgewählt ist aus der Gruppe bestehend aus Biopolymeren, Peptiden, Proteinen und Sacchariden.

Im Rahmen der vorliegenden Anmeldung wird ein solches Implantat als "Polymer-Implantat" bezeichnet. Ein solches Polymer-Implantat ist ein medizinisches Implantat im Sinne der vorliegenden Erfindung.

Der Begriff Polymer" im Sinne der vorliegenden Erfindung umfasst sowohl synthetisch als auch biologisch vorkommenden Polymere. Beispielsweise kann das Polymer Saccharide umfassen.

In einer bevorzugten Ausführungsform ist das Polymer ausgewählt aus der Gruppe bestehend aus Sacchariden, vorzugsweise Hyaluronsäure, Alginat, Dextran, Chitosan, Chitin, Heparin, Heparansulfat, Dermatansulfat, Chondoitinsulfat und/oder deren Derivate, besonders bevorzugt Hyaluronsäure, Alginat, Dextran, Chitosan, Chitin, Heparin, Heparansulfat, und synthetischen Polymeren, vorzugsweise Poly(lactid-co-glycolid), Polylactid, Polyglykolid, Poly(β-hydroxybutyrat), Poly(p-dioxanon), Polyester, Polyesteramid, Polyvinylalkohol, Polycaprolacton eingesetzt werden. Als nicht degradable Polymere werden vorzugsweise Polyethylen, Polyethylenglykol, Polypropylen, Polyethylenterephthalat, Polyethylenglykol, Polymethylmethacrylat, Polyphenylenoxid, Polysulfon, Polycarbonat, Poly-2-Hydroxyethylmethacrylat, Polyurethan, Polyvinyl-N-Pyrrolidon, Polysiloxan oder Polyacrylamid eingesetzt.

Das Molekulargewicht der Polysaccharide liegt vorzugsweise zwischen 250000 - 1000000 g/mol.

Beispiele geeigneter Hyaluronsäure-Derivate sind in Figur 4 dargestellt.

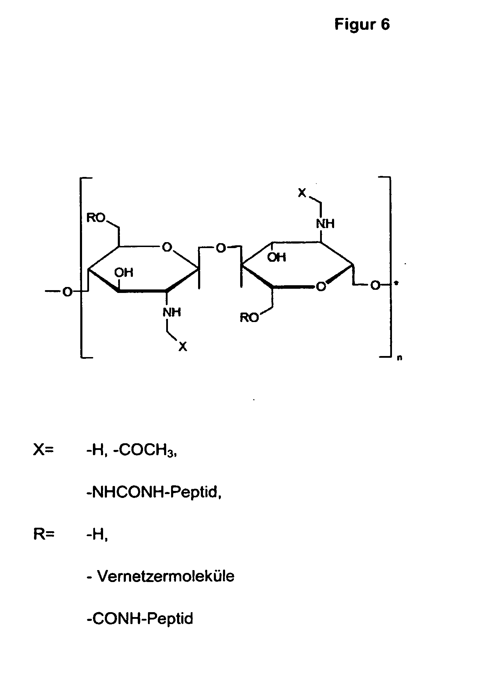

Beispiele geeigneter Chitin-Derivate sind in Figur 6 dargestellt.

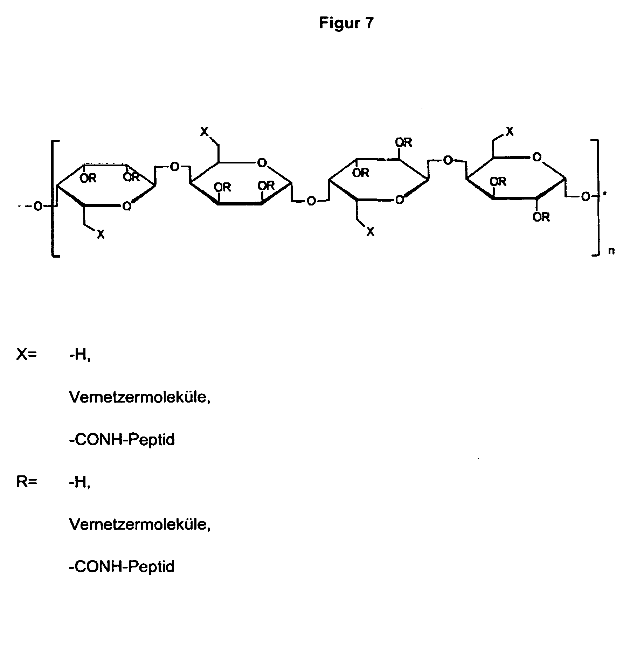

Beispiele geeigneter Alginat-Derivate sind in Figur 7 dargestellt.

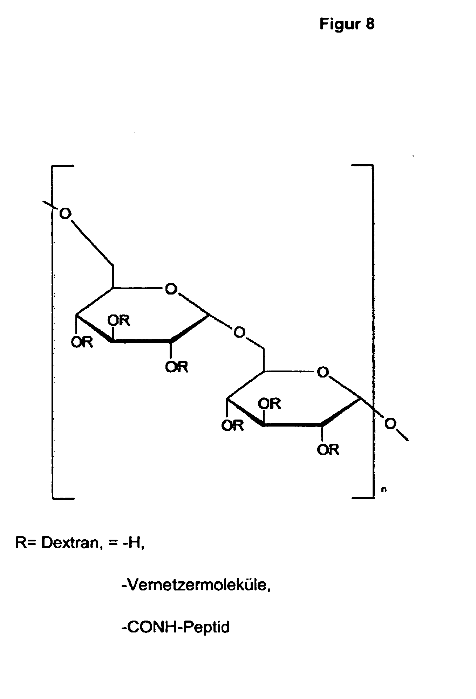

Beispiele geeigneter Dextran-Derivate sind in Figur 8 dargestellt.

Der Begriff "kontaktiert" im Sinne der vorliegenden Erfindung beschreibt mindestens eine Wechselwirkung des proximalen Endes des Polymers mit dem Oberflächenbereich des Kerns.

In einer bevorzugten Ausführungsform handelt es sich bei dieser mindestens einen Wechselwirkung um eine unmittelbare Wechselwirkung.

"Unmittelbare Wechselwirkung" im Sinne der vorliegenden Erfindung bedeutet, dass zwischen dem Polymer kein weiteres Molekül liegt.

Diese unmittelbare Wechselwirkung umfasst jede Art von chemischer Wechselwirkung, beispielsweise um lonenbindung, um metallische Bindungen, um Atombindung, um Komplexbindung, um Dipol-Dipol-Wechselwirkung (beispielsweise Wasserstoffbrückenbindungen), um Dipol-Ion-Wechselwirkung, um Van-der-Waals-Wechselwirkungen.

In einer bevorzugten Ausführungsformen, handelt es sich bei der unmittelbaren Wechselwirkung jedoch ausschließlich um nicht-kovalente Wechselwirkungen.

In einer Vielzahl von Anwendungen ist es von Vorteil, eine möglichst wasserunlösliche und hydrolyseunempfindliche Polymerschicht zu erhalten.

In einer besonderen Ausführungsform ist das Polymer-Implantat daher dadurch gekennzeichnet, dass das Implantat mehrere Linker Lₚ umfasst, die miteinander vernetzt sind.

Zur Herstellung einer wasserunlöslichen und hydrolyseunempfindlichen Polymerschicht können die Polymere mit einer Vielzahl an chemischen Reaktionen vernetzt werden.

Verschiedene Moleküle, beispielsweise Hexamethylendiisocyanat, Dialdehyd-Verbindungen wie Glutaraldehyd, Divinylsulfon, Diepoxy-Verbindungen wie Ethylenglykoldiglycidylehter, Epichlorhydrin, Dihydrazide wie Oktandihydrazid, Carbodiimide, Methacrylsäureanhydrid, Mercaptopropionsäure, können für die Vernetzung eingesetzt werden.

Solcherlei Moleküle werden als Vernetzermoleküle bezeichnet.

Dem Fachmann sind weitere geeignete Vernetzermoleküle bekannt. Auch ist der Fachmann in der Lage durch einfache Versuche weitere geeignete Vernetzermoleküle zu ermitteln.

Auch im erfindungsgemäßen Polymer-Implantat kann das Ausmaß und die Verteilung des Bereichs der Oberfläche, welche mit dem Linker Lₚ kontaktiert wird, sowie die Dichte der Kopplung, variieren.

In einer Ausführungsform kontaktiert der Linker Lp im Wesentlichen nur den Teil der Oberfläche, welcher zur Verankerung in dem Gewebe, vorzugsweise dem Gingiva-Gewebe, vorgesehen ist.

Auch die Dichte der Linker Lₚ, die die Oberfläche kontaktieren kann variieren.

Erfindungsgemäß kann im Polymer-Implantat das Biomolekül auf verschiedene Art und Weise gebunden sein.

In einer Ausführungsform ist das Polymer-Implantat dadurch gekennzeichnet, dass zwischen dem Biomolekül und dem proximalen Ende des Linkers Lp durchgehend kovalente Bindungen vorliegen.

In einer anderen Ausführungsform ist das Polymer-Implantat dadurch gekennzeichnet, dass zwischen dem Biomolekül und dem proximalen Ende des Linkers Lp wenigstens eine nicht-kovalente Bindung mit einer Assoziationskonstante von mindestens 10⁸ M⁻¹, bevorzugter mindestens 10⁹ M⁻¹, noch bevorzugter mindestens 10¹⁰ M⁻¹, besonders bevorzugt mindestens 10¹² M⁻¹, vorliegt.

In einer bevorzugten Ausführungsform hiervon ist das Polymer-Implantat dadurch gekennzeichnet, dass die wenigstens eine nicht-kovalente Bindung durch (jeweils) eine Biotin-Komponente und einen Biotin-Bindungspartner gebildet wird.

In einer Ausführungsform dieser bevorzugten Ausführungsform ist eine Biotin-Komponente kovalent an das distale Ende des Linkers Lₚ gebunden. Diese Biotin-Komponente ist nicht-kovalent an einen Biotin-Bindungspartner gebunden. Dieser Bindungspartner ist außerdem nicht-kovalent an eine Biotin-Komponente gebunden, welche kovalent an das Biomolekül gebunden ist.

In einer anderen Ausführungsform dieser bevorzugten Ausführungsform ist ein Biotin-Bindungspartner kovalent an den Linker Lp gebunden. Dieser Biotin-Bindungspartner ist nicht-kovalent an einen Biotin-Komponente gebunden, welche kovalent an das Biomolekül gebunden ist.

Das erfindungsgemäße Implantat umfasst einen Kern.

Der Begriff "Kern" im Sinne der vorliegenden Erfindung ist der innere Teil des Implantats, der mindestens in seinem Oberflächenbereich einen im Wesentlichen nach außen geschlossenen, d.h. im Wesentlichen nicht von innen nach außen durchbrochenen, Bereich enthält. Der Kern weist ein gewisses Maß an Härte auf.

Vorzugsweise weist er eine Härte von mindestens 6, vorzugsweise mindestens 7, besonders bevorzugt mindestens 8, ganz besonders bevorzugt mindestens 9 Härtegraden auf der Mohs-Skala auf.

In bestimmten Ausführungsformen ist der Kern einphasig, ist also ein Formkörper aus einer im Wesentlichen homogenen Masse.

In anderen Ausführungsformen ist er mehrphasig. Er kann aus mehreren Schichten bestehen. Auch möglich ist, dass innerhalb des Kerns verschiedene Konzentrationsgradienten von Bestandteilen des Kerns zu finden sind.

Der Begriff "Oberflächenbereich" im Sinne der vorliegenden Erfindung ist der den Kern nach außen abgrenzende Bereich. Bei einphasigen Kernen ist der Oberflächenbereich im Wesentlichen nicht durch seine Zusammensetzung von weiter innen liegenden Bereichen des Kerns abgrenzbar.

Bei mehrphasigen Kernen, beispielsweise bei Kernen mit einem schichtartigen Aufbau, ist der Oberflächenbereich durch seine Morphologie von weiter innenliegenden Bereichen anderer Schichten abgrenzbar.

Die Schichtdicke des Oberflächenbereichs beträgt vorzugsweise mindestens 1 µm, besonders bevorzugt mindestens 2 µm, ganz besonders bevorzugt mindestens 10 µm.

Der Begriff "Oberfläche" im Sinne der vorliegenden Erfindung bezeichnet die nach außen gewandte Fläche des Oberflächenbereichs.

Der Begriff "anorganisches Material" im Sinne der Erfindung umfasst jedes Material, dass für den Oberflächenbereich von Implantaten wie sie in der Medizin verwendet werden geeignet und ggf. zugelassen sind. Solche anorganischen Materialien sind dem Fachmann bekannt. Dazu zählen beispielsweise Keramiken und Metalle bzw. Metallverbindungen. Als Keramiken kommen beispielsweise Zirkonoxidkeramiken, Aluminiumoxidkeramiken in Betracht. Als Metalle finden beispielsweise Gold, Platin und Titan bzw. Verbindungen und/oder Legierungen enthaltend diese Metalle bzw. deren Verbindungen Verwendung.

In einer bevorzugten Ausführungsform umfasst der Oberflächenbereich des Implantats Titan und/oder ein Titanoxid.

Als Titanoxide kommen Titanoxid, Titandioxid oder andere Titanoxide der allgemeinen Formel TiOₓ, wobei x im Bereich von 0 bis 2 liegt, in Betracht.

Bevorzugter Weise ist der Oberflächenbereich des Implantats umfassend Titan und/oder ein Titanoxid im Wesentlichen frei von Gold oder Gold-Ionen.

Das erfindungsgemäße Implantat umfasst ein Biomolekül.

Bevorzugt umfasst das erfindungsgemäße Implantat mehrere verschiedene Biomoleküle. Biomoleküle können auf schonende Weise kovalent an Biotin-Komponenten B₂ gebunden werden, ohne dass sich die ggf. native Struktur der Biomoleküle unter den dafür erforderlichen Reaktionsbedingungen ändert. Zahlreiche mit Biotin verknüpfte Biomoleküle sind kommerziell erhältlich. Erfindungsgemäß können Kombinationen bzw. Mischungen verschiedener Biomoleküle gleichzeitig und nicht-kovalent an das erfindungsgemäße Implantat gebunden werden, wodurch eine Oberfläche geschaffen werden kann, welche einer natürlichen Umgebung näher kommt als Oberflächen, welche lediglich mit einer einzelnen Art Biomoleküle belegt sind. Die Anlagerung der Biomoleküle an die Oberfläche des Implantats erfolgt über die Biotin-Komponente B₂ und ist somit hochselektiv, stabil und besonders schonend.

Der Begriff "Biomolekül" im Sinne der vorliegenden Erfindung umfasst jedes organische Molekül, welches für das Einwachsen des Implantats in das Gewebe förderlich ist.

Biomoleküle im Sinne der Erfindung sind biokompatibel, d.h. mit biologischen Systemen gut verträglich. Häufig sind biokompatible Substanzen biologischen Ursprungs.

In einer bevorzugten Ausführungsform umfasst der Begriff "Biomolekül" im Wesentlichen die Zelladhäsion fördernde Biomoleküle, d.h. das Biomolekül oder die Biomoleküle sind für das Einwachsen oder Anwachsen des umliegenden Gewebes in das Implantat förderlich. In einer anderen bevorzugten Ausführungsform umfasst der Begriff "Biomolekül" im Wesentlichen die Zelladhäsion hindernde Biomoleküle. Geeignete Vertreter beider Gruppen von Biomolekülen sind dem Fachmann bekannt.

Ob ein Molekül für das Einwachsen des Gewebes in das Implantat förderlich ist, kann der Fachmann durch bekannte Testverfahren in einfachen Versuchen bestimmen.

In einer bevorzugten Ausführungsform beträgt die Dichte des Biomoleküls auf der Oberfläche des Implantats oder einer Teiloberfläche des Implantats wenigstens 10 fmol/cm², bevorzugter wenigstens 100 fmol/cm², noch bevorzugter wenigstens 1 pmol/cm², am bevorzugtesten wenigstens 10 pmol/cm² und insbesondere wenigstens 100 pmol/cm². Die Dichte des Biomoleküls kann je nach Art und Anwendung deutlich höher liegen, z.B. 20 nmol/cm² betragen.

Vorzugsweise ist das Biomolekül ausgewählt aus der Gruppe bestehend aus Biopolymeren, Peptiden, Proteinen und Sacchariden.

Der Begriff "Biopolymere" im Sinne der vorliegenden Erfindung umfasst alle Moleküle, die eine Protein-Grundstruktur enthalten. Diese Protein-Grundstruktur ist vorzugsweise aus den 20 natürlich vorkommenden Aminosäuren aufgebaut. Bei der Protein-Grundstruktur kann es sich um natürlich vorkommender Proteine oder Fragmente davon handeln. Solche Fragmente eines Proteins können verschieden lang sein, beispielsweise mindestens 20 Aminosäuren oder mindestens 30 Aminosäuren (soweit das entsprechende Proteine mindestens so lang ist) oder mindestens 40 Aminosäuren (soweit das entsprechende Proteine mindestens so lang ist) oder mindestens 50 Aminosäuren (soweit das entsprechende Proteine mindestens so lang ist) oder mindestens 60 Aminosäuren (soweit das entsprechende Proteine mindestens so lang ist).

Fragmente, die kürzer als 20 Aminosäuren sind werden im Sinne der vorliegenden Erfindung als Peptide bezeichnet.

Es kann sich auch um Proteine handeln, die eine Homologie von mindestens 70 Prozent, bevorzugt mindestens 80 Prozent, noch bevorzugter mindestens 90 Prozent, besonders bevorzugt mindestens 95 Prozent, ganz besonders bevorzugt mindestens 98 Prozent zu einem natürlich vorkommenden Protein oder eines Fragmentes eines solchen natürlich vorkommenden Protein aufweisen.

Neben dieser Protein-Grundstruktur kann das Biomolekül verschiedene Seitenketten enthalten. Es kann beispielsweise glykosyliert, acetyliert, alkyliert, biotinyliert, formyliert, carboxyliert, glutaminyliert, hydroxyliert, prenyliert, farnesyliert, myristoyliert, palmitoyliert, pegyliert, phosphoryliert werden oder beispielsweise mit Nukleotiden, Phosphatidylinositol gekoppelt werden.

Bei diesen Seitenketten kann es sich um in natürlichen Proteinen vorkommenden Seitenketten handeln oder auch um andere Seitenketten. Auch kann das Molekül neben der genannten Protein-Grundstruktur weitere Aminosäuren-enthaltende Ketten enthalten.

Der Fachmann ist in der Lage, auf Grundlage verschiedener bekannter Testverfahren durch einfache Versuche zu bestimmen, welche dieser Moleküle zur Verwendung im erfindungsgemäßen Implantat geeignet sind.

Vorzugsweise handelt es sich bei dem Biopolymer um ein Biopolymer welches eine Komponente der Extrazellulären Matrix des Gewebes im Sinne der vorliegenden Erfindung ist oder ein Homolog, Fragment oder Variante einer solchen Komponente.

Der Homologiegrad des Homologs bzw. des Fragments bzw. der Variante beträgt vorzugsweise mindestens 70 Prozent, bevorzugt mindestens 80 Prozent, noch bevorzugter mindestens 90 Prozent, besonders bevorzugt mindestens 95 Prozent, ganz besonders bevorzugt mindestens 98 Prozent.

Besonders bevorzugt ist das Biopolymer ausgewählt aus der Gruppe bestehend aus Adhäsionsproteinen, vorzugsweise Fibronektin, Vitronektion, Fibrinogen, Tenascin oder Thrombospondin; Wachstumsfaktoren, vorzugsweise aus der Familie der *Bone Morphogenic Proteins* oder *Fibroblast Growth Factor,* Matrixproteinen, vorzugsweise Kollagen.

Ganz besonders bevorzugt ist Fibronektin oder eine Mischung, welche neben zumindest einem weiteren Biomolekül Fibronektin enthält.

In einer besonderen Ausführungsform ist das erfindungsgemäße Implantat daher dadurch gekennzeichnet, dass das Biomolekül ausgewählt ist aus der Gruppe bestehend aus Adhäsionsproteinen, vorzugsweise Fibronektin, Vitronektion, Fibrinogen, Tenascin oder Thrombospondin; Wachstumsfaktoren, vorzugsweise Bone Morphogenic Protein oder Fibroblast Growth Factor; Matrixproteinen, vorzugsweise Kollagen.

Adhäsionsmoleküle wie Fibronektin sind bekannt für ihre vielfältige Wirkung im Organismus. Durch Interaktion mit Integrinen und anderen Rezeptormolekülen bewirken sie unterschiedlichste Reaktionen in den gebundenen Zellen wie Differenzierung, Adhäsion, Proliferation und weitere Zellmechanismen. Die Anwendung zur gezielten Einwirkung auf verschiedene Zelltypen wie Osteoblasten, Chrondrozyten oder Fibroblasten ist möglich. Fibronektin ist ein ca. 520.000 Da großes, dimeres Glykoprotein. Es sind jedoch auch Fibronektion-Varianten beispielsweise kommerziell erhältlich, die ein anderes Molekulargewicht, beispielsweise ca. 440 kDa umfassen. Beide Untereinheiten des Proteins besitzen eine nahezu identische Struktur. Als Bestandteil des Bindegewebes hat es vor allem eine zentrale Rolle als Linkerprotein zwischen der EZM und den hier angesiedelten Zellen fördert so die Zelladhäsion. Dies geschieht über eine Verknüpfung zwischen den Kollagenfibrillen der Matrix, in die die Fibronektinmoleküle eingelagert sind, und den Integrinen, welche an Zelloberflächen lokalisiert sind. Gebildet wird Fibronektin im Extrazellularraum von Fibroblasten, welche ihre Adhäsion somit selbstständig initiieren. Das Protein besteht aus drei sich wiederholenden Modultypen (Modultyp 1 - 3) wobei Modultyp 3 mit 15 - 17 Wiederholungen pro Untereinheit das häufigste in Fibronektin vorkommende Modul ist. Weiterhin ist es das mit ca. 90 Aminosäuren größte und gleichzeitig wichtigste Modul. Es enthält einen kurzen Sequenzabschnitt bestehend aus den drei Aminosäuren: Arginin, Glycin und Asparaginsäure (RGD-Sequenz). Diese zyklische Sequenz bindet an eine Signalsequenz der membranständigen Integrine und bewirkt so die Verankerung der Fibroblasten in der EZM. Das Tripeptid RGD wurde 1984 von Pierschbacher und Ruoslahti als minimale essenzielle Zelladhäsionssequenz in Fibronektin entdeckt und als Bindungsepitop für Integrine identifiziert. Es konnte gezeigt werden, dass immobilisierte RGD-Peptide ähnlich wie ECM-Proteine integrinvermittelte Zelladhäsion bewirken, während lösliche RGD-Peptide antagonistisch zur ECM adhärierte Zellen ablösen.

Kollagen als Bestandteil der EZM tritt ebenfalls wie oben erwähnt mit diesen Adhäsionsmolekülen in Kontakt und sorgt für eine Anbindung und Präsentation zur Interaktion mit membrangebundenen Rezeptoren. Kollagen ist ein vernetztes Biopolymer, das in vielen Variationen im Organismus auftritt und sich daher gut als Beschichtungsgrundlage eignet. Kollagen ist das häufigste Protein im Organismus überhaupt. Es besteht aus drei umeinander gewickelten Einzelkollagenpeptidketten. Diese Trippelhelix mit einem Durchmesser von 1,5 nm fügt sich zu dickeren Kollagenfibrillen mit einem Durchmesser von 10- 300 nm, je nach Typ, zusammen. Hieraus bilden sich wiederum Kollagenfasern mit einer Dicke von 0,5 - 3 µm. Kollagen bestimmt die Zugfestigkeit und den Charakter des Bindegewebes. Dies geschieht zum einem über die Konzentration der Kollagenfasern und zum anderen über deren Anordnung.

Andere geeignete Bestandteile der Extrazellulären Matrix sind beispielsweise die Laminine.

Alle diese Biopolymere können auf vielerlei Weise miteinander und mit anderen Biomolekülen kombiniert werden.

Der Begriff "Peptid" im Sinne der vorliegenden Erfindung umfasst Moleküle umfassend eine Aminosäurekette von bis zu 19 Aminosäuren.

Auch die Peptide können, analog den Biopolymeren, mit Seitenketten modifiziert werden.

Insbesondere umfasst der Begriff Peptide-Sequenzen, die mit den im Gewebe vorhandenen Integrinen, vorzugsweise den α₅β₁-Integrinen, wechselwirken. Beispiele solcher Peptid-Sequenzen sind RGD-, RGDS-, GRGD-, GRGDS-, GRGDY-, YRGDG-, GRGDSP-, GRGDSY-, GRGDSPK-, CGRGDSY-, RGDSPASSKP-, und Cyclo(RGDfK).

In einer besonderen Ausführungsform ist das erfindungsgemäße Implantat daher dadurch gekennzeichnet, dass das Biomolekül ein Peptid ist ausgewählt ist aus der Gruppe bestehend aus RGD-, RGDS-, GRGD-, GRGDS-, GRGDY-, YRGDG-, GRGDSP-, GRGDSY-, GRGDSPK-, CGRGDSY-, RGDSPASSKP-, und Cyclo(RGDfK).

In einer bevorzugten Ausführungsform beträgt die Dichte 10 fmol Peptid/cm² - 20,5 nmol/cm².

Alle diese Peptide können auf vielerlei Weise miteinander und mit anderen Biomolekülen kombiniert werden.

Damit die Peptid-Sequenzen ihren Zweck optimal erfüllen, ist es von Vorteil die Peptide in einem gewissen Abstand zu Oberfläche zu immobilisieren. Dies kann durch den Einsatz von "Abstandhalte"-Molekülen gewährleistet werden. Die Länge des "Abstandhalte"-Moleküls liegt vorzugsweise zwischen 11-46 Å. Als "Abstandhalte"-Molekül können sowohl hydrophile als auch hydrophope Ketten eingesetzt werden. Beispielsweise können sich wiederholende Aminohexansäure-, Glycin-Einheiten oder Oligo(Ethylenglykole) verwendet werden.

Neben linearen "Abstandhalte"-Molekül-Ketten und nur einem gekoppelten Peptid, besteht die Möglichkeit eine verzweigte "Abstandhalte"-Molekül-Kette mit mehreren gekoppelten Peptiden einzusetzen. Oder eine verzeigte "Abstandhalte"-Molekül-Kette mit auch nur einem gekoppelten Peptid.

Der Begriff "Saccharid" im Sinne der vorliegenden Erfindung umfasst sowohl Mono- als auch Disaccharide als auch Polysaccharide.

In einer bevorzugten Ausführungsform ist das Saccharid ausgewählt aus der Gruppe bestehend aus Hyaluronsäure, Heparin, Heparinsulfat, Dermatansulfat, Chondroitinsulfat, Alginat, Dextran, Chitosan und Chitin.

In einer besonderen Ausführungsform ist das erfindungsgemäße Implantat daher dadurch gekennzeichnet, dass das Saccharid ausgewählt ist aus der Gruppe bestehend aus Hyaluronsäure, Heparin, Heparinsulfat, Dermatansulfat, Chondroitinsulfat, Alginat, Dextran, Chitosan und Chitin.

Ein weitere Bestandteil der EZM sind Glycosaminoglycanen (GAGs). GAGs sind lineare aus sich wiederholenden Disacchariden aufgebaute, saure Polysaccharide. Sie besitzen die Fähigkeit Wasser zu speichern und gewährleisten, dass das Gewebe eine gewisse Druckfestigkeit aufweist. Zu den GAGs gehören Hyaluronsäure, Heparin, Heparinsulfat, Dermatansulfat und Chondroitisulfat. Sie sind biokompatibel und biodegradabel und neben der Stabilisierung und Organisation des Gewebes regulieren sie die Zelladhäsion, Zellproliferation und Differenzierung. Infolgedessen findet Hyaluronsäure (HA) und ihre Derivate ein weites Anwendungsfeld im medizinischen Bereich.

Hyaluronsäure ist ein einfaches GAG der EZM und kommt als einziges GAG nicht als Proteoclykan vor. Es wird an der Oberfläche der Fibroblasten synthetisiert und lässt sich ferner auch biotechnologisch gewinnen. Hyaluronsäure ist eine hochmolekulare Verbindung mit einem Molekulargewicht zwischen 50000 und mehreren Millionen Dalton. Der Grundbaustein ist ein aus D-Glucuronsäure und N-Acetyl-D-Glucosamin in β-1,3-glykosidischer Bindung aufgebautes Aminodisaccharid, das untereinander β-1,4-glykosidisch verbunden ist. Hyaluronsäure kommt in Gelenken, dem Glaskörper des Auges, in der Haut vor, ist also ein körpereigenes Produkt und interagiert mit Zelloberflächenrezeptoren. Die bekanntesten der Rezeptoren sind der CD44 Rezeptor und Rhamm (*Receptor hyaluronic acid mediated motility*)*.* Darüber hinaus wurden noch eine große Anzahl weiterer Bindungsproteine aufgefunden, deren Funktion jedoch nur teilweise bekannt ist. Die Hyaluronsäure-bindenden Zellrezeptoren leiten Informationen über verschiedene Signaltransduktionswege, die die Regulation von Proteinkinasen und GTPasen einschließen, zum Zytoskelett und beeinflussen so das Wachstum der Zellen. Das Hyaluronsäuremolekül weist verschiedene funktionelle Gruppen auf, die über chemischen Umsetzungen modifiziert werden können. Es sind die Carboxylgruppen, die Hydroxylgruppen, die sekundäre Aminogruppen und nach Deacetylierung auch die primären Aminogruppen, die für chemische Derivatisierung zugänglich sind. Eine weitere Möglichkeit bestehen in der Aufspaltung der Zuckerringe z.B. durch oxydative Agentien. In der Literatur sind viele Modifizierungsmöglichkeiten zu finden (Bulpitt P., J. Biomed. Mater Res 47(2), 1999, 152-69; Luo Y, J Control Release, 69(1), 2000, 169-84; Vercruysse KP, Bioconjug. Chem., 8(5), 1997, 686-94; T. Segura, Biomaterials 26, 2005, 359-371). Die Spaltung der Hyaluronsäure erfolgt durch Hyaluronidasen nach einem Hydrolyse- und durch die Hyaluronatlyase nach einem Eliminierungs-Mechanismus. Um die möglicherweise zu schnelle Degradation auf der modifizierten Implantatoberfläche zu verhindern bzw. zu verlangsamen, können die Hyaluronsäure und ihre Derivate durch Quervernetzung gegenüber dem Abbauenzym stabilisiert werden. Hierzu wird auf die in der Literatur zahlreich beschriebenen Verfahren verwiesen. Die Vernetzungsreaktionen sind Gegenstand vieler Patente und werden unter anderem in den Patenten US5510121, WO09802204 und EP161887 beschreiben.

Alle diese Saccharide können auf vielerlei Weise miteinander und mit anderen Biomolekülen kombiniert werden.

Dem Fachmann ist bekannt, dass sich die genaue Zusammensetzung der Extrazellulären Matrix in einzelnen Geweben unterscheiden kann. Expressionsmuster von Bestandteilen der Extrazellulären Matrix sind in einschlägigen Datenbanken und in der Fachliteratur beschrieben.

Dementsprechend ist der Fachmann in der Lage, gegebenenfalls, das Spektrum der Biomoleküle in einer gegebenen Ausführungsform des erfindungsgemäßen Implantats so anzupassen, dass es möglichst schnell in das entsprechende Gewebe einwachsen kann, was dazu beitragen kann, das Risiko einer Infektion am Ort der Implantation zu verringern.

Bevorzugt umfasst das erfindungsgemäße Implantat einen Oberflächenbereich, an den eine Mischung von Biomolekülen gebunden ist, vorzugsweise Fibronektin und wenigstens ein weiteres Biomolekül. In einer bevorzugten Ausführungsform ist das weitere Biomolekül ausgewählt aus der Familie der BMP (*Bone Morphogenic Proteins*). In diesem Fall ist die betreffende Oberfläche besonders gut für das Anwachsen von Osteoblasten geeignet. In einer anderen bevorzugten Ausführungsform ist das weitere Biomolekül ausgewählt aus der Famile der FGS (*Fibroblast Growth Factors*). In diesem Fall ist die betreffende Oberfläche besonders gut für das Anwachsen von Fibroblasten geeignet.

In einer bevorzugten Ausführungsform kombiniert das erfindungsgemäße Implantat Oberflächenbereiche, welche besonders gut für das Anwachsen von Fibroblasten geeignet sind, mit Oberflächenbereichen, welche besonders gut für das Anwachsen von Osteoblasten geeignet sind. Die besondere Eignung der betreffenden Oberflächenbereiche für den jeweiligen Zweck kann durch Wahl geeigneter Biomoleküle oder deren Mischungen erreicht werden, welche mit Hilfe der Erfindung besonders einfach und schonend an die Oberfläche des Implantats gebunden werden können.

Dem Fachmann sind geeignete Verfahren bekannt, mit denen gewährleistet wird, dass bestimmte Oberflächenbereiche mit bestimmten Biomolekülen oder deren Mischungen belegt sind, und andere nicht. In diesem Zusammenhang kann beispielsweise auf Maskentechniken, z.B. lithographische Techniken, verwiesen werden. Es ist jedoch auch möglich, dass die einzelnen Komponenten zunächst separat unterschiedlich beschichtet werden und erst danach zum eigentlichen Implantat zusammengefügt werden.

Diese Ausführungsform ist in besonderem Maße für Dentalimplantate geeignet. Diese Dentalimplantate umfassen üblicherweise einen unteren Bereich, z.B. ein Gewinde, welcher zur Verankerung des Implantats im Kiefer vorgesehen ist, einen mittleren Bereich, z.B. eine ringartige Struktur am oberen Ende des unteren Bereichs, welcher in Kontakt mit dem Zahnfleisch kommt, und einem oberen Bereich, z.B. eine künstliche Zahnkrone oder einen Adapter, auf den eine solche Zahnkrone aufgesetzt werden kann. In einer bevorzugten Ausführungsform ist die Oberfläche des mittleren Bereichs eines solchen Dentalimplantats besonders gut für das Anwachsen von Fibroblasten geeignet, was z.B. durch Biomoleküle ausgewählt aus Fibronektin und FGS oder deren Mischungen erreicht werden kann. In einer anderen bevorzugten Ausführungsform ist die Oberfläche des unteren Bereichs eines solchen Dentalimplantats besonders gut für das Anwachsen von Osteoblasten geeignet, was z.B. durch Biomoleküle ausgewählt aus Fibronektin und BMP oder deren Mischungen erreicht werden kann. In einer besonders bevorzugten Ausführungsform sind sowohl der untere Bereich als auch der mittlere Berich entsprechend hergerichtet.

Auch kann ggf. von Vorteil sein, das Biomolekül bzw. das Spektrum der in einem Implantat verwendeten Biomoleküle spezifisch auf die Situation in einem bestimmten Patienten anzupassen. Gründe hierfür können beispielsweise bestimmte Allergien eines Patienten sein oder ein individuelles Expressionsmuster der EZM-Moleküle am Ort der Implantation.

Eine solche maßgeschneiderte Beschichtung einer Vorstufe des erfindungsgemäßen Implantats ist aufgrund des erfindungsgemäßen Schichtsystems leicht möglich.

Weiterhin können beispielsweise Antikörper oder Teile von Antikörpern gegen Bestandteile der Extrazellulären Matrix als Biomoleküle verwendet werden.

Als strukturbildende Komponente kann biotinyliertes Kollagen gebunden werden, in das die genannten Adhäsionsproteine und/oder Wachstumsfaktoren unspezifisch eingelagert werden können.

Der Aufbau begünstigt die native Faltung der Biomoleküle, was der Funktion zu Gute kommt. Der Abstand zwischen Oberfläche und dem Biomolekül ist variierbar. Durch die Verwendung körpereigener Substanzen, wie Kollagen und Hyaluronsäure, sowie körperverträgliche Stoffe, wie Streptavidin, genügt der Aufbau des Schichtsystems in hohem Maße der Anforderung der Biokompatibilität.

Unter bestimmten Umständen ist nicht nur die Adhäsion von Zellen an Implantatoberflächen und damit das Einwachsen des Implantates in das Gewebe gewünscht, sondern das genaue Gegenteil, nämlich ein Verhindern des Gewebeanwachsens an das Implantat.

Beispielsweise soll verhindert werden, dass neu eingesetzte Stents sich durch anwachsendes Gewebe wieder verschließen und es so zu einer Gefäßrestinose kommt. Weiterhin verringert sich die Lebensdauer von eingesetzten Kathetern im menschlichen Organismus, wenn sie von Gewebe zugewuchert werden. Solche Katheter müssen operativ ersetzt werden, was eine starke Belastung für den Patienten darstellt.

Nicht zuletzt zu erwähnen sind auch hier Dentalimplantate. Zwar soll an diesen in bestimmten Bereichen des Implantats das Anwachsen des Gewebes (vor allem des Weichgewebes) gefördert werden, allerdings nicht bei allen Stellen des Implantates gleichstark. Wuchert beispielsweise die Gingiva während des Einheilprozesses in den Knochen, bewirkt dies in den meisten Fällen ein Lockern des Implantates im Kieferknochen und somit eine verkürzte Lebensdauer des eingesetzten Implantates.

Dies sind nur einige Punkte, die zeigen, dass das Verhindern des Anwachsens von Gewebe an Implantaten ebenfalls ein sehr wichtiger Aspekt der Implantologie darstellt. Dem Anwachsen von Gewebe geht immer eine Adhäsion von Zellen voraus. Dieser wiederum geht die Anbindung von Proteinen, welche die Zelladhäsion fördern oder erst ermöglichen, voraus.

Das Haupt-Target zur Verhinderung der Gewebeadhäsion stellt somit das Verhindern des Anbindens von adhäsionsfördernden Proteinen dar. Dies kann wie folgt erreicht werden:

Streptavidin ist für seine Bindung zu Biotin bekannt, welche auch als die stärkste nicht kovalente Wechselwirkung in der Natur mit einer Zerfallskonstante von KON = 10¹⁵ beschrieben wird. Aufgrund dieser Starken Wechselwirkung wird es auch zur Beschichtung von Oberflächen eingesetzt, um über die zuvor beschriebene Wechselwirkung spezifisch (biologische) Moleküle an einer Oberfläche zu immobilisieren.

Diese Funktion als "Haftvermittler" ergibt sich durch die Anordnung der Biotinbindungsstellen im Protein. Hier liegen jeweils zwei gegenüber, wodurch zwei genutzt werden können, um an zuvor mit Biotin modifizierte Oberflächen zu binden, und die beiden verbleibenden, um anschließend weitere biotinylierte Proteine an der Streptavidinmonolage spezifisch zu immobilisieren.

Es wurde nun überraschend festgestellt, dass diese Monolage eine weitere interessante Eigenschaft besitzt: Sie unterbindet das unspezifische Anbinden von unbiotinylierten Proteinen an Oberflächen.

Besonders interessant ist diese Eigenschaft im Hinblick auf eine Inhibierung des Anbindens von adhäsionsfördernden Proteinen, allen voran Fibronektin, das wichtigste Protein im Bereich der Zellanbindung. Es interagiert mit den Integrinen in Zellmembranen und bindet Zellen so *in vivo* an die Extrazelluläre Matrix (EZM).

Es konnte durch SPR-Messungen gezeigt werden, dass Fibronektin spontan und ohne weitere äußere Einflüsse unspezifisch auf TiO₂ Oberflächen adsorbiert (5,8 nm mittlere optische Schichtdicke). Zwar besitzen die immobilisierten Proteine nicht mehr dieselbe Aktivität wie sie bei einer spezifischen Immobilisierung besäßen, dennoch reicht diese Aktivität bereits aus, um eine Zelladhäsion zu fördern und somit den ersten Schritt für eine Gewebeadhäsion an Titanimplantate.

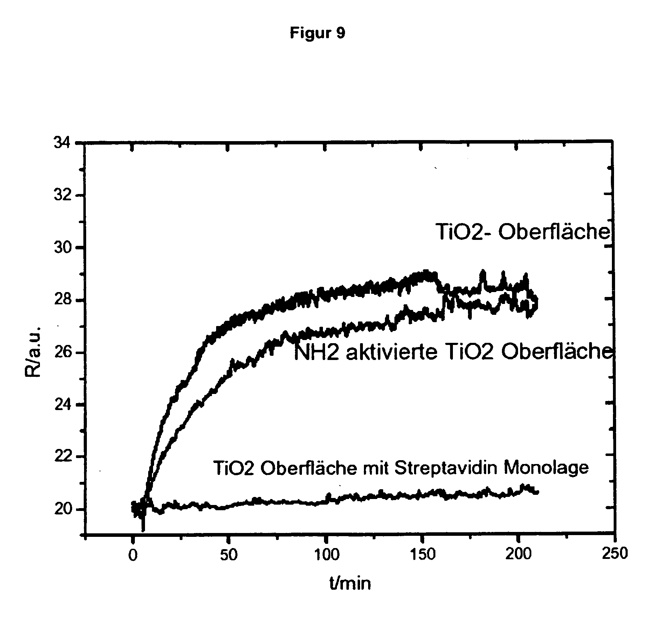

Fibronektin bildet sogar auf aminoaktivierten Titanoberflächen Schichten mit einer vergleichbaren Dichte aus (6 nm). Die Bindung von Fibronektin ist sehr stark und lässt sich auch durch starkes Spülen nicht mehr lösen. Bringt man allerdings eine Streptavidinmonolage auf eine Titanoberfläche, bzw. auf eine aminoaktiverte Titanoberfläche auf, so adsorbiert Fibronektin praktisch nicht mehr an dieser Oberfläche und gebundene Proteine lassen sich nach dem Spülvorgang fast restlos wieder entfernen (0.4 nm mittlere optische Schichtdicke). Ein entsprechender vergleich der Fibronektinadsorptionskinetiken ist in Figur 9 dargestellt (es wurde jeweils nach 200 Minuten gespült).

Somit wird die Möglichkeit eröffnet, eine Anbindung von adhäsionsfördernden Proteinen an Implantatoberflächen aus Titan (oder anderen Materialien) komplett zu unterbinden. Damit ist ebenfalls das Anbinden von Bindegewebszellen unterbunden und damit ebenfalls das Anwachsen von Gewebe an solche Oberflächen.

Außerdem stellt die vorliegende Erfindung ein Verfahren zur Herstellung eines erfindungsgemäßen Implantats zur Verfügung. Das erfindungsgemäße Verfahren ist dadurch gekennzeichnet, dass der Kern mit dem Biomolekül zusammengebracht wird.

Je nach spezifischer Ausführungsform des erfindungsgemäßen Implantats kann sich das Verfahren unterscheiden.

Beispielhaft sind in der vorliegenden Anmeldung Verfahrensschritte aufgeführt. Der Fachmann ist in der Lage, diese durch weitere notwendige Schritte zu ergänzen bzw. diese hinsichtlich der Herstellung spezifischer Ausführungsformen anzupassen.

Die verschiedenen erfindungsgemäßen Implantate können in einer Vielzahl von Geweben für eine Vielzahl von therapeutischen Anwendungen verwendet werden.

Diese Anwendungen der verschiedenen Ausführungsformen sind dem Fachmann bekannt.

Somit umfasst die vorliegende Erfindung die Verwendung eines Biomoleküls im Sinne der vorliegenden Erfindung zur Herstellung eines erfindungsgemäßen Implantats.

In einer bevorzugten Ausführungsform des erfindungsgemäßen Implantats handelt es sich um Dentalimplantate. Diese werden zur Behandlung einer Vielzahl von Schäden, Verletzungen etc. des Dentalbereichs verwendet. Insbesondere werden sie zur Behandlung von Zahnschäden verwendet.

Der Begriff "Zahnschäden" im Sinne der vorliegenden Erfindung umfasst den Verlust eines oder mehrerer, vorzugsweise nebeneinander liegender, Zähne, oder auch andere Schädigungen der Zähne wie ein Abbrechen einzelner Teile, das Auseinanderbrechen des Zahnes, Entfernung von Teilen des Zahnes.

Hierunter fallen beispielsweise künstliche Zahnwurzeln, die zur Befestigung von Kronen, Brücken oder Prothesen dienen, Vollimplantate, aber auch jede andere Form von Dentalimplantaten.

Somit umfasst die vorliegende Erfindung die Verwendung eines Biomoleküls zur Herstellung eines erfindungsgemäßen Implantats zur Behandlung von Zahnschäden.

Die nachfolgenden Beispiele illustrieren die Erfindung, ohne dass sie diese beschränken sollen.

### Beispiele

### 1. Herstellung eines Implantats mit kovalent gebundenem Linker und via Streptavidin-Biotin-Wechselwirkung verbundenem Fibronektin

### Methode I

Das Titansubstrat wird für ca. 60 Minuten in einer 0,5 mM N-(6-Aminohexyl)aminopropyl-trimethox-aminosilan, gelöst in Methanol, aktiviert. Anschließend wird das Substrat entnommen, gründlich mit Methanol und Ethanol gereinigt und für ca. 220 Minuten in einer 0,25 mM Lösung des Succinimid-Derivates Sulfo-NHS-LCLC-Biotin, gelöst in Ethanol, inkubiert. Anschließend wird das Substrat entnommen, gründlich mit dem Lösemittel und PBS-Puffer abgespült und in einer 0,5 µM Streptavidin Lösung, gelöst in PBS-Puffer, für ca. eine Stunde inkubiert. Das Substrat wird im Anschluss gründlich mit PBS-Puffer gereinigt und für 200 Minuten bei Beibehalten des Lösemittels in einer 25 nM Lösung aus biotinyliertem Fibronektin inkubiert. Abschließend wird das Substrat noch mit viel PBS-Puffer gründlich gereinigt.

### Methode II

Das Titansubstrat wird für ca. 60 Minuten in einer 0,5 mM N-(6-Aminohexyl)aminopropyl-trimethox-aminosilan, gelöst in Methanol, aktiviert. Anschließend wird das Substrat entnommen, gründlich mit Methanol und PBS-Puffer gereinigt und für ca. 120 Minuten in einer 0,5 mM Lösung des Succinimid-Derivates Sulfo-NHS-LC-Biotin, gelöst in destilliertem H₂O, inkubiert. Anschließend wird das Substrat entnommen, gründlich mit PBS-Puffer abgespült und in einer 0,5 µM Streptavidin Lösung, gelöst in PBS-Puffer, für ca. eine Stunde inkubiert. Das Substrat wird im Anschluss gründlich mit PBS-Puffer gereinigt und für 200 Minuten bei Beibehalten des Lösemittels in einer 25 nM Lösung aus biotinyliertem Fibronektin inkubiert. Abschließend wird das Substrat noch mit viel PBS-Puffer gründlich gereinigt.

Biotinylierung: Humanes Fibronektin wird in hochreinem Wasser in einer Endkonzentration von 1 mg/ml gelöst. Sulfo-NHS-Biotin, Sulfo-NHS-LC-Biotin oder Sulfo-NHS-LC-LC-Biotin werden ebenfalls kurz vor Reaktion in hochreinem Wasser in Konzentrationen von maximal 0,5 mg/ml gelöst. Fibronektin und Biotinylierungsreagenz werden im Verhältnis von 1:100 zusammengegeben und auf eine Endkonzentration von 25 nM mit PBS (pH 7,4) eingestellt. Mischung bei 4 °C 2 Stunden reagieren lassen und anschließend aufreinigen.

### 2. Herstellung von Implantaten mit einem Linker umfassend ein Polymer (Polymer-Implantat)

### A) Immobilisierung von Hyaluronsäure mit RGD-Peptid auf Titan

### Vorbehandlung:

Vor der Modifizierung mit dem entsprechendem Polymer wird die Titandioxidschicht mit einem organischen Lösungsmittel (Aceton, Ethanol) entfettet, anschließend mit Wasser gewaschen und mit einem Stickstoffstrom getrocknet. Die Beschichtung erfolgt mittels Schleuder- oder Tauchbeschichtung. Die Schicht soll zwischen 1µm und 500µm dick sein.

### HA Vernetzung mit einem Diepoxid

Hyaluronsäure (HA) (MW 1x10⁶*) mit einer Konzentration von 10 mg/ml wird in einer 0,5% NaOH-Lösung suspendiert. Nachdem sich das Polymer komplett gelöst hat wird 1,4-Butandiol-diglycidylether (BDDE) dazugegeben. Pro 1 mg HA werden 0,5µl BDDE dazugegeben. Der Reaktionsprozess dauert 16 h und kann gegebenenfalls wiederholt werden. Unreagiertes BDDE wird mittels Diafiltration gegen Wasser entfernt. Die Probe kann dann auf die gewünschte Konzentration aufkonzentriert werden und bei -20°C gefriergetrocknet werden.

### HA Bioaktivierung mit (Aminohexansäure)3-cyclo(RGDfK)-Peptid

Das Peptid, bestehend aus fünf Aminosäuren und einer sich drei Mal wiederholendem Aminohexansäure-Molekülsubstruktur, wird nach der Standard-Festphasenpeptidsynthese nach Merrifield hergestellt. Das Peptid wird kovalent an die zuvor hergestellten reaktiven Aldehyd-Gruppen der HA gebunden. Die bereits vernetzte HA (2mg) wird in einer 100mM Natriumacetat-Lösung (pH=5,6) hydratisiert. Dazu werden eine 500mM Natriumperjodat-Lösung, das Peptid (20mg/ml in Natriumacetat-Puffer) und eine 50mM Natriumcyanoborohydrid-Lösung gegeben. Das Reaktionsendvolumen beträgt 2ml auf 2mg vernetzte HA. Die Reaktion läuft für 4h bei RT. Anschließen wird 5-mal mit einer Salzlösung mit niedrigem pH-Wert (1% Essigsäure/ 1M NaCl) gewaschen, um ionisch gebundenes Peptid zu lösen. Danach wird mit destilliertem Wasser gewaschen und gefriergetrocknet.

### B) Immobilisierung von biotinylierter Hyaluronsäure mit Fibronektin auf Streptavidin

### Vorbehandlung:

Vor der Modifizierung mit dem entsprechendem Polymer wird die Titandioxidschicht mit einem organischen Lösungsmittel (Aceton, Ethanol) entfettet, anschließend mit Wasser gewaschen und mit einem Stickstoffstrom getrocknet. Die Beschichtung erfolgt mittels Schleuder- oder Tauchbeschichtung. Die Schicht soll zwischen 1µm und 500µm dick sein.

### Biotinylierung von Hyaluronsäure

Natriumhyaluronat (500mg, 1,25mmol) wird in Wasser gelöst (50ml). EDC (1-Ethyl-3-(3-dimethylaminopropyl)carbodiimid) (74,3mg, 0,623mmol) und Sulfo-NHS (N-hydroxysulfosuccinimide) (134mg, 0,623mmol) werden als Festsoff zur HA-Lösung gegeben und gelöst. Erst danach wir AAD (Adipinsäuredihydrazid) (6,5g, 37,5 mmol) dazugegeben. Die Reaktion läuft für 16h unter rühren bei RT. Nach Abschluss der Reaktion wird HA-AAD mittel Dialyse (gegen 150mM NaCl) gereinigt. Das Produkt wird danach gefriergetrocknet. Biotin wird in Form von Sulfo-NHS-LC-Biotin (Sulfosuccinimidyl-6-(biotinamido)hexanoate) in das HA-AAD eingebunden. Es werden 100mg Sulfo-NHS-LC-Biotin zu einer HA-AAD-Lösung (300mg, 3mg/ml in PBS-Puffer) dazu gegeben. Die Reaktion läuft für 16h unter rühren bei RT. Anschließend wird mittels Dialyse gegen Wasser für 24h aufgereinigt. Das gereinigte Produkt wird gefriergetrocknet und bei 4°C gelagert.

### Vernetzung der biotinylierten HA

1,4-Butandiol-diglycidylether (BDDE) (theoretisch 100%ige Verlinkung: 2 mol BDDE für 1 mol HA) wird zu HA-Biotin (3%w/v in 0,1N NaOH) gegeben und verrührt. Die Reaktion ist nach 45 min bei einer Temperatur 60°C abgeschlossen. Danach wir mit Eisessig (70µl) neutralisiert. Das Gel kann anschließend auf einer Scheibe ausgegossen werden und für 2-3 Tage getrocknet werde.

### Streptavidin-Immobilisierung des biotinylierten HA-Gels

Die trockene biotinylierte HA wird in einer Streptavidin-Lösung (1µM in PBS-Puffer) für ca. 1h hydratisiert. Anschließend wird das Gel drei Mal mit PBS Puffer gewaschen, um unspezifisch gebundenes Streptavidin zu entfernen. Die Lagerung erfolgt hydratisiert in PBS-Puffer bei 4°C.

### Fibronektin-Immobiliseirung des Streptavidin-HA-Gels

Das hydratisierte Streptavidin-HA-Gel wird auf RT aufgewärmt und überstehender PBS-Puffer wird mit einer Spritze abgezogen. Es wir eine 5*10-8 Molare Fibronektin-Lösung (bereits biotinyliert; Biotinylierungsgrad = 10) in PBS-Puffer angesetzt. Die Lösung (2 ml) wird zu dem Streptavidin-HA-Gel dazugegeben. Die Inkubationszeit soll 200 Minuten nicht unterschreiten. Nach Inkubation wird drei Mal mit PBS-Puffer gewaschen. Die Lagerung erfolgt hydratisiert in PBS-Puffer bei 4°C.

### C) Immobilisierung eines vernetzten HA-Kollagen-Gels auf Titan

### Vorbehandlung:

Vor der Modifizierung mit dem entsprechendem Polymer wird die Titandioxidschicht mit einem organischen Lösungsmittel (Aceton, Ethanol) entfettet, anschließend mit Wasser gewaschen und mit einem Stickstoffstrom getrocknet. Die Beschichtung erfolgt mittels Schleuder- oder Tauchbeschichtung. Die Schicht soll zwischen 1µm und 500µm dick sein.

### Synthese eines vernetzten HA-Kollagen-Gels

1,4-Butandiol-diglycidylether (BDDE) (theoretisch 100%ige Verlinkung: 2 mol BDDE für 1 mol HA) wird zu HA (3%w/v in 0,1N NaOH) gegeben und verrührt. Danach wir mit Eisessig (70µl) neutralisiert. Anschließend wird Kollagen in einer Konzentration von 1 mg/ml dazugegeben und durchmischt. Anschließend wir die modifizierte, vernetzte HA 2-3 Tage bei Raumtemperatur getrocknet.

## Patentansprüche

1. Medizinisches Implantat umfassend einen Kern, mindestens einen Linker L₁, mindestens eine Biotin-Komponente B₁, mindestens einen Biotin-Bindungspartner, mindestens eine Biotin-Komponente B₂, und mindestens ein Biomolekül, wobei
- der Linker L₁ ein proximales Ende und ein distales Ende umfasst, mit dem proximalen Ende kovalent an den Kern und mit dem distalen Ende kovalent an die Biotin-Komponente B₁ gebunden ist, wobei zwischen der Bindung des Linkers L₁ an den Kern und der Bindung des Linkers L₁ an die Biotin-Komponente B₁ durchgehend kovalente Bindungen vorliegen;
- die Biotin-Komponente B₂ kovalent mit dem Biomolekül verbunden ist; und
- der Biotin-Bindungspartner wenigstens eine Bindungsstelle S₁ für die Biotin-Komponente B₁ aufweist, an welche die Biotin-Komponente B₁ nicht-kovalent gebunden ist, und wenigstens eine Bindungsstelle S₂ für die Biotin-Komponente B₂ aufweist, an welche die Biotin-Komponente B₂ nicht-kovalent gebunden ist;
wobei das Biomolekül ausgewählt ist aus der Gruppe bestehend aus Biopolymeren, Peptiden und Sacchariden.

2. Implantat nach Anspruch 1, **dadurch gekennzeichnet, dass** die Biotin-Komponente B₁ an die Bindungsstelle S₁ des Biotin-Bindungspartners und/oder die Biotin-Komponente B₂ an die Bindungsstelle S₂ des Biotin-Bindungspartners über eine nicht-kovalente Wechselwirkung mit einer Assoziationskonstante von mindestens 10⁸ M⁻¹ gebunden ist.

3. Implantat nach Anspruch 1 oder 2, **dadurch gekennzeichnet, dass** der Biotin-Bindungspartner ausgewählt ist aus der Gruppe bestehend aus Avidin-, Neutravidin-und Streptavidin-Komponenten.

4. Implantat nach einem der vorstehenden Ansprüche, **dadurch gekennzeichnet, dass** zwei Biotin-Komponenten B₁ an zwei Bindungsstellen S₁ eines einzelnen Biotin-Bindungspartners gebunden sind.

5. Implantat nach einem der vorstehenden Ansprüche, **dadurch gekennzeichnet, dass** das Biomolekül zwei jeweils kovalent gebundene Biotin-Komponenten B₂ umfasst, welche jeweils nicht-kovalent an Biotin-Bindungspartner gebunden sind.

6. Implantat nach einem der vorstehenden Ansprüche, **dadurch gekennzeichnet, dass** es eine Vielzahl von Biotin-Komponenten B₁ und eine Vielzahl von Biotin-Bindungspartnern mit Bindungsstellen S₁ und S₂ umfasst, wobei die Biotin-Komponenten B₁ jeweils über Linker L₁ kovalent an den Kern gebunden sind und zumindest ein Anteil der Biotin-Komponenten B₁ nicht-kovalent an Bindungsstellen S₁ gleicher oder verschiedener Biotin-Bindungspartner gebunden ist.

7. Implantat nach Anspruch 6, **dadurch gekennzeichnet, dass** es mindestens einen Bereich umfasst, in dem die Flächenkonzentration der Gesamtheit aller Biotin-Komponenten B₁ im Bereich von 1 - 2 Biotin-Komponenten B₁ pro 24 nm² liegt.

8. Implantat nach Anspruch 6 oder 7, **dadurch gekennzeichnet, dass** das relative molare Verhältnis von Biotin-Bindungspartnern zu Biotin-Komponenten B₁ im Bereich von 1 : 2 bis 1 : 4 liegt.

9. Implantat nach einem der vorstehenden Ansprüche, **dadurch gekennzeichnet, dass** die Biotin-Komponente einen Spacer umfasst, welcher kovalent an das distale Ende des Linkers L₁ gebunden ist.

10. Implantat nach Anspruch 9, **dadurch gekennzeichnet, dass** es
- Linker L₁ vom Typ L₁' enthält, welche nicht an Biotin-Komponenten B₁ gebunden sind, und
- Linker L₁ vom Typ L₁" enthält, welche kovalent an Biotin-Komponenten B₁ gebunden sind, wobei der mittlere Abstand der distalen Enden der Linker L₁" zum Schwefelatom der Biotin-Komponenten B₁ 1,6 nm beträgt.

11. Implantat nach einem der vorstehenden Ansprüche, **dadurch gekennzeichnet, dass** es verschiedene Oberflächenbereiche mit einer Mindestfläche von jeweils 1,0 mm² umfasst, an welche verschiedene Biomoleküle oder verschiedene Mischungen unterschiedlicher Biomoleküle gebunden sind.

12. Implantat nach einem der vorstehenden Ansprüche, **dadurch gekennzeichnet, dass** das proximale Ende des Linkers L₁ über mindestens ein Si- oder P-Atom an den Kern gebunden ist.

13. Implantat nach Anspruch 12, **dadurch gekennzeichnet, dass** das Si- oder P-Atom Teil einer funktionellen Gruppe ist ausgewählt aus Silanen, Siloxanen, Phosphaten, Phosphonaten und Phosphoranen.

14. Verwendung eines Biomoleküls zur Herstellung eines Implantats nach einem der Ansprüche 1 bis 13 zur Behandlung von Zahnschäden.

## Claims

1. Medical implant comprising a core, at least one linker L₁, at least one biotin component B₁, at least one biotin binding partner, at least one biotin component B₂ and at least one biomolecule, where
- the linker L₁ comprises a proximal end and a distal end, with the proximal end being covalently bonded to the core and with the distal end being covalently bonded to the biotin component B₁, where continuous covalent bonds are present between the binding of the linker L₁ to the core and the binding of the linker L₁ to the biotin component B₁;
- the biotin component B₂ is covalently bonded to the biomolecule; and
- the biotin binding partner has at least one binding site S₁ for the biotin component B₁ to which the biotin component B₁ is non-covalently bonded, and has at least one binding site S₂ for the biotin component B₂ to which the biotin component B₂ is non-covalently bonded;
where the biomolecule is selected from the group consisting of biopolymers, peptides and saccharides.

2. Implant according to Claim 1, **characterized in that** the biotin component B₁ is bonded to the binding site S₁ of the biotin binding partner and/or the biotin component B₂ at the binding site S₂ of the biotin binding partner via a non-covalent interaction with an association constant of at least 10⁸ M⁻¹.

3. Implant according to Claim 1 or 2, **characterized in that** the biotin binding partner is selected from the group consisting of avidin, neutravidin and streptavidin components.

4. Implant according to one of the preceding claims, **characterized in that** two biotin components B₁ are bonded to two binding sites S₁ of a single biotin binding partner.

5. Implant according to one of the preceding claims, **characterized in that** the biomolecule comprises two in each case covalently bonded biotin components B₂ which are in each case bonded non-covalently to biotin binding partners.

6. Implant according to one of the preceding claims, **characterized in that** it comprises a large number of biotin components B₁ and a large number of biotin binding partners with binding sites S₁ and S₂, where the biotin components B₁ are in each case bonded covalently to the core via linkers L₁, and at least a proportion of the biotin components B₁ is bonded non-covalently to binding sites S₁ of identical or different biotin binding partners.

7. Implant according to Claim 6, **characterized in that** it comprises at least one region in which the concentration per area of the totality of all biotin components B₁ is in the range from 1-2 biotin components B₁ per 24 nm².

8. Implant according to Claim 6 or 7, **characterized in that** the relative molar ratio of biotin binding partners to biotin components B₁ is in the range from 1:2 to 1:4.

9. Implant according to one of the preceding claims, **characterized in that** the biotin component comprises a spacer which is covalently bonded to the distal end of the linker L₁.

10. Implant according to Claim 9, **characterized in that** it comprises
- linkers L₁ of the type L₁' which are not bonded to biotin components B₁, and
- linkers L₁ of the type L₁" which are covalently bonded to biotin components B₁, where the average distance between the distal ends of the linkers L₁" and the sulphur atom of the biotin components B₁ is 1.6 nm.

11. Implant according to one of the preceding claims, **characterized in that** it comprises different surface regions with a minimum area of in each case 1.0 mm² onto which are bonded various biomolecules or various mixtures of different biomolecules.

12. Implant according to one of the preceding claims, **characterized in that** the proximal end of the linker L₁ is bonded to the core via at least one Si or P atom.

13. Implant according to Claim 12, **characterized in that** the Si or P atom moiety of a functional group is selected from silanes, siloxanes, phosphates, phosphonates and phosphoranes.

14. Use of a biomolecule for producing an implant according to one of Claims 1 to 13 for treating dental decay.

## Revendications

1. Implant médical comprenant un noyau, au moins un lieur L₁, au moins un composant de type biotine B₁, au moins un partenaire de liaison de la biotine, au moins un composant de type biotine B₂ et au moins une biomolécule
- le lieur L₁ comprenant une extrémité proximale et une extrémité distale et étant lié avec l'extrémité proximale par covalence au noyau et avec l'extrémité distale par covalence au composant de type biotine B₁, des liaisons covalentes se situant sans interruption entre la liaison du lieur L₁ au noyau et la liaison du lieur L₁ au composant de type biotine B₁ ;
- le composant de type biotine B₂ étant lié par covalence à la biomolécule ; et
- le partenaire de liaison de la biotine présentant au moins un site de liaison S₁ pour le composant de type biotine B₁, auquel le composant de type biotine B₁ est lié de manière non covalente, et au moins un site de liaison S₂ pour le composant de type biotine B₂, auquel le composant de type biotine B₂ est lié de manière non covalente ;
la biomolécule étant choisie dans le groupe constitué par les biopolymères, les peptides et les saccharides.

2. Implant selon la revendication 1, **caractérisé en ce que** le composant de type biotine B₁ est lié au site de liaison S₁ du partenaire de liaison de la biotine et/ou le composant de type biotine B₂ est lié au site de liaison S₂ du partenaire de liaison de la biotine via une interaction non covalente, présentant une constante d'association d'au moins 10⁸ M⁻¹.

3. Implant selon la revendication 1 ou 2, **caractérisé en ce que** le partenaire de liaison de la biotine est choisi dans le groupe constitué par les composants de type avidine, neutravidine et streptavidine.

4. Implant selon l'une quelconque des revendications précédentes, **caractérisé en ce que** deux composants de type biotine B₁ sont liés à deux sites de liaison S₁ d'un seul partenaire de liaison de la biotine.

5. Implant selon l'une quelconque des revendications précédentes, **caractérisé en ce que** la biomolécule comprend deux composants de type biotine B₂ à chaque fois liés par covalence, qui sont à chaque fois liés de manière non covalente au partenaire de liaison de la biotine.

6. Implant selon l'une quelconque des revendications précédentes, **caractérisé en ce qu'**il comprend une multitude de composants de type biotine B₁ et une multitude de partenaires de liaison de la biotine présentant des sites de liaison S₁ et S₂, les composants de type biotine B₁ étant à chaque fois liés via des lieurs L₁ par covalence au noyau et au moins une partie des composants de type biotine B₁ étant liés de manière non covalente aux sites de liaison S₁ de partenaires de liaison de la biotine identiques ou différents.

7. Implant selon la revendication 6, **caractérisé en ce qu'**il comprend au moins une zone, dans laquelle la concentration de surface de la totalité de tous les composants de type biotine B₁ se situe dans la plage de 1-2 composants de type biotine B₁ par 24 nm².

8. Implant selon la revendication 6 ou 7, **caractérisé en ce que** le rapport molaire relatif de partenaires de liaison de la biotine aux composants de type biotine B₁ se situe dans la plage de 1:2 à 1:4.

9. Implant selon l'une quelconque des revendications précédentes, **caractérisé en ce que** le composant de type biotine comprend un écarteur, qui est lié par covalence à l'extrémité distale du lieur L₁.

10. Implant selon la revendication 9, **caractérisé en ce qu'**il
- contient des lieurs L₁ du type L₁', qui ne sont pas liés à des composants de type biotine B₁, et
- contient des lieurs L₁ du type L₁", qui sont liés par covalence aux composants de type biotine B₁, la distance moyenne entre les extrémités distales du lieur L₁" et l'atome de soufre des composants de type biotine B₁ étant de 1,6 nm.

11. Implant selon l'une quelconque des revendications précédentes, **caractérisé en ce qu'**il comprend différentes zones de surface présentant une surface minimale à chaque fois de 1,0 mm², auxquelles différentes biomolécules ou différents mélanges de différentes biomolécules sont liés.

12. Implant selon l'une quelconque des revendications précédentes, **caractérisé en ce que** l'extrémité proximale du lieur L₁ est liée via au moins un atome de Si ou de P au noyau.

13. Implant selon la revendication 12, **caractérisé en ce que** l'atome de Si ou de P fait partie d'un groupe fonctionnel choisi parmi les silanes, les siloxanes, les phosphates, les phosphonates et les phosphoranes.

14. Utilisation d'une biomolécule pour la préparation d'un implant selon l'une quelconque des revendications 1 à 13 pour le traitement de dommages dentaires.
